# EUROPEAN PATENT APPLICATION

(11) **EP 4 180 454 A1**
(43) Date of publication of application: **17.05.2023**
(21) Application number: 21836979.1
(22) Date of filing: 09.07.2021
(51) Int. Cl.: C07K 16/00, A61K 39/395, C12N 15/13

(54) **ANTI-IGE ENGINEERED ANTIBODY AND APPLICATION THEREOF**

(30) Priority: 10.07.2020 CN 202010663029; 01.07.2021 CN 202110750959
(71) Applicant: Shanghai Jemincare Pharmaceuticals Co., Ltd., Pudong New Area, Shanghai 201203 (CN); Jiangxi Jemincare Group Co., Ltd., Nanchang, Jiangxi 330096 (CN)
(72) Inventor: DENG, Sujun, Shanghai 201203 (CN); GU, Chunyin, Shanghai 201203 (CN); LIU, Xiaowu, Shanghai 201203 (CN); CAO, Xiaodan, Shanghai 201203 (CN); LIU, Peipei, Shanghai 201203 (CN); ZHANG, Jianjian, Shanghai 201203 (CN); PAN, Zhongzong, Shanghai 201203 (CN); XIAO, Zheng, Shanghai 201203 (CN); WANG, Xueping, Shanghai 201203 (CN); GUO, Haibing, Shanghai 201203 (CN)
(74) Representative: Bianchetti & Minoja SRL
(86) International application number: PCT/CN2021/105620
(87) International publication number: WO 2022/007965

(57) **Abstract**

The present application relates to an antigen-binding protein comprising amino acid mutations compared with the light and heavy chain variable region sequences of omalizumab. The antigen-binding protein of the present application is an anti-IgE engineered antibody. The present application also relates to pharmaceutical compositions comprising the antigen-binding protein, and methods thereof for alleviation or treatment of diseases associated with abnormal level of IgE.

## Description

### Technical field

The present application relates to the field of biomedicine, in particular to an anti-IgE engineered antibody and a use thereof in diseases with abnormal level of IgE.

### Background

Immunoglobulin E (IgE) is a protein with a molecular weight of 180KD. Although IgE only accounts for 0.002% of total serum Ig, it is closely related to the pathological process of allergic diseases. In different tissues and organs, increaed level of IgE can cause inflammatory immune responses, the most common of which are allergic asthma, allergic rhinitis and sinusitis, allergic conjunctivitis, systemic allergies, skin allergies and urticaria, or the like.

In recent years, biological agents such as anti-IgE monoclonal antibodies have become a research hotspot in the treatment of diseases related to abnormal level of IgE. In 2003, the anti-IgE antibody omalizumab was first approved by the FDA for the treatment of asthma. In 2014, the U.S. Food and Drug Administration (FDA) approved omalizumab for the treatment of chronic idiopathic urticaria. The application of omalizumab has the following problems: (1) it targets free IgE but does not (or cannot effectively) target pathogenic species of the IgE/ FcεRI (IgE Fc receptor) complex at pharmaceutically relevant doses; (2) its dosing concentration and interval are directly related to a patient's body weight and level of free IgE, such that when the patient's body weight and level of free IgE exceed a certain range, its dosing concentration as well as frequency will increase, which increases a complexity and inconvenience for control and management of the pateint's disease; (3) its affinity with IgE is not particularly good.

Therefore, there is an urgent need for new engineered anti-IgE antibodies with improved efficacy and compliance.

### SUMMARY OF THE INVENTION

The present application provides an antigen-binding protein, which has been carried out engineering optimization on the antibody light and heavy chain sequences of omalizumab, and can include at least one of the following features: (1) compared to omalizumab, the humanization degree is improved, the probability of production of anti-drug antibody is reduced, and the loss of potential efficacy is reduced; (2) post-translational modification sites are mutated and chemical stability (for example, the stability under high temperature or pH environment) is improved; (3) the affinity with human IgE is improved by yeast display method, the drug efficacy is improved, and it is expected that the antigen-binding protein can be applied to patients with higher body weight or higher concentration of free IgE; (4) it binds monkey IgE with high affinity, which is helpful for the pharmacodynamic study in preclinical animal models; (5) it effectively blocks the binding of IgE to IgE Fc receptors (Fc ε RI); (6) it's serum half-life is prolonged, which can reduce the frequency of injections and facilitate the control of the patient's condition.

In one aspect, the application provides an antigen-binding protein, the antigen-binding protein comprises an antibody light chain variable region (VL), wherein compared with the amino acid sequence as shown in SEQ ID NO: 22, the VL comprises amino acid mutations at one or more positions selected from the group consisting of: E55 and V104.

In some embodiments, the VL comprises amino acid mutations at one or more positions selected from the group consisting of D30b, V29, D30, Y30a, and G30c.

In some embodiments, the VL comprises amino acid mutations at positions of the following group: D30b, E55 and V104.

In some embodiments, the VL comprises amino acid mutations at positions selected from the group consisting of (1) D30b, E55, V104, V29, D30, and Y30a; (2) D30b, E55, V104, D30, Y30a and G30c; (3) D30b, E55, V104 and Y30a; (4) D30b, E55, V104, Y30a and G30c; (5) D30b, E55, V104, D30, Y30a and G30c; and (6) D30b, E55, V104, D30 and Y30a.

In some embodiments, the amino acid mutation at position E55 comprises E55Q.

In some embodiments, the amino acid mutation at position V104 comprises V104L.

In some embodiments, the amino acid mutation at position D30b comprises D30bE.

In some embodiments, the amino acid mutation at position V29 comprises V29L.

In some embodiments, the amino acid mutation at position Y30a comprises any one selected from the group consisting of Y30A, Y30aS, and Y30aD.

In some embodiments, the amino acid mutation at position D30 comprises any one selected from the group consisting of D30Y, D30A, D30E, D30F, D30G, and D30N.

In some embodiments, the amino acid mutation at position G30c comprises any one selected from the group consisting of G30cA, G30cY, and G30cW.

In some embodiments, the VL comprises the following group of amino acid mutations: D30bE, E55Q and V104L.

In some embodiments, the VL comprises the amino acid sequence as shown in SEQ ID NO: 57.

In some embodiments, the VL comprises the any one of amino acid sequence as shown in SEQ ID NOs: 23-34.

In some embodiments, the antigen-binding protein comprises an antibody heavy chain variable region (VH), wherein compared with the amino acid sequence as shown in SEQ ID NO: 50, the VH comprises amino acid mutations at one or more positions selected from the group consisting of 137, A49, S50 and D54.

In some embodiments, the VH comprises amino acid mutations at one or more positions selected from the group consisting of N60, P61, 167, T30, S31, S96 and H97.

In some embodiments, the VH comprises amino acid mutations at positions of the following group: N60, P61, I67, I37, A49, S50, and D54.

In some embodiments, the amino acid mutation at position 137 comprises I37V.

In some embodiments, the amino acid mutation at position A49 comprises A49S.

In some embodiments, the amino acid mutation at position S50 comprises S50V.

In some embodiments, the amino acid mutation at position D54 comprises D54A.

In some embodiments, the amino acid mutation at position N60 comprises N60A.

In some embodiments, the amino acid mutation at position P61 comprises P61D.

In some embodiments, the amino acid mutation at position 167 comprises 167F.

In some embodiments, the amino acid mutation at position T30 comprises T30R.

In some embodiments, the amino acid mutation at position S31 comprises S31Q.

In some embodiments, the amino acid mutation at position S96 comprises S96T.

In some embodiments, the amino acid mutation at position H97 comprises H97N.

In some embodiments, the VH comprises the following group of amino acid mutations: N60A, P61D, I67F, I37V, A49S, S50V, and D54A.

In some embodiments, the VH comprises the amino acid sequence as shown in SEQ ID NO: 58.

In some embodiments, the VH comprises any one of amino acid sequences as shown in SEQ ID NOs: 51-56.

In some embodiments, the VL includes amino acid mutations at the following positions: D30b, E55, and V104; and the VH includes amino acid mutations at the following positions: N60, P61, I67, I37, A49, S50, and D54.

In some embodiments, the VL and the VH comprise amino acid mutations at positions selected from the group consisting of: (1) VL comprises amino acid mutations at the following positions: D30b, E55, V104, V29, D30, and Y30a; VH includes amino acid mutations at the following positions: N60, P61, I67, I37, A49, S50, D54, S96 and H97; (2)

VL includes amino acid mutations at the following positions: D30b, E55, V104, D30, Y30a and G30c; VH includes amino acid mutations at the following positions: N60, P61, I67, I37, A49, S50, D54, and T30; (3) VL includes amino acid mutations at the following positions: D30b, E55, V104, and Y30a; VH includes the following amino acid mutations at positions: N60, P61, I67, I37, A49, S50, D54 and S96; (4) VL includes amino acid mutations at the following positions: D30b, E55, V104 and Y30a; VH includes amino acid mutations at the following positions: N60, P61, I67, I37, A49, S50 and D54 (5) VL includes amino acid mutations at the following positions: D30b, E55, V104, Y30a and G30c; VH includes amino acid mutations at the following positions: N60, P61, I67, I37, A49, S50 and D54 (6) VL includes amino acid mutations at the following positions: D30b, E55, V104 and Y30a; VH includes amino acid mutations at the following positions: N60, P61, I67, I37, A49, S50, D54 and H97; (7) VL includes amino acid mutations at the following positions: D30b, E55, V104, D30, Y30a and G30c; VH includes amino acid mutations at the following positions: N60, P61, I67, I37, A49, S50 and D54 (8) VL includes amino acid mutations at the following positions: D30b, E55, V104, D30 and Y30a; VH includes amino acid mutations at the following positions: N60, P61, 167, 137, A49, S50, D54 and H97; (9)

VL includes amino acid mutations at the following positions: D30b, E55, V104, D30, Y30a and G30c; VH includes amino acid mutations at the following positions: N60, P61, I67, I37, A49, S50, D54 and S96; and (10) VL includes amino acid mutations at the following positions: D30b, E55, V104, D30, Y30a and G30c; VH includes amino acid mutations at the following positions: N60, P61, I67, I37, A49, S50, D54 and S31.

In some embodiments, the antigen-binding protein comprises an antibody heavy chain constant region.

In some embodiments, the antibody heavy chain constant region of the antigen-binding protein is derived from a human IgG constant region.

In some embodiments, the human IgG constant region comprises a human IgG1 constant region.

In some embodiments, the human IgG1 constant region comprises one or more amino acid mutations selected from the group consisting of M252Y, S254T and T256E.

In some embodiments, the human IgG1 constant region comprises any one of amino acid sequence as shown in SEQ ID NO: s: 60-61.

In some embodiments, the antigen-binding protein comprises an antibody light chain constant region.

In some embodiments, the antibody light chain constant region comprises the amino acid sequence as shown in SEQ ID NO: 59.

In another aspect, the application provides one or more isolated nucleic acid molecules encoding the antigen-binding proteins described herein.

In another aspect, the application provides vectors comprising the nucleic acid molecules described herein.

In another aspect, the application provides cells comprising the nucleic acid molecule or the vector described herein.

In another aspect, the present application provides a method for preparing the antigen-binding protein described herein, the method comprising culturing the cells under conditions allowing the expression of the antigen-binding protein.

In another aspect, the application provides a composition comprising the antigen-binding protein, the nucleic acid molecules, the vector and/or the cells, and optionally a pharmaceutically acceptable vector.

On the other hand, the present application provides a use of the antigen-binding protein, the nucleic acid molecules, the vector, the cells and/or the composition in prepartion of a medicament for alleviating or treating a disease associated with abnormal level of IgE.

In another aspect, the application provides a method of alleviating or treating a disease associated with abnormal level of IgE, the method comprising administering the antigen-binding protein, the nucleic acid molecules, the vector, the cells and/or the composition to a subject in need thereof.

Other aspects and advantages of the present application can be readily appreciated by those skilled in the art from the following detailed description. Only exemplary embodiments of the present application are shown and described in the following detailed description. As those skilled in the art will recognize, the content of this application enables those skilled in the art to make changes to the specific embodiments disclosed without departing from the spirit and scope of the invention to which this application relates. Accordingly, the drawings and descriptions in the specification of the present application are only exemplary and not restrictive.

### Description of drawings

The particularity features of the invention to which this application relates is set forth in the claims appended. The features and advantages of the invention involved in this application can be better understood by reference to the exemplary embodiments described in detail hereinafter and the accompanying drawings. A brief description of the drawings is as follows.
Figure 1 shows that inhibition rates of free IgE in cynomolgus monkey serum by omalizumab, AB1904AM10 and AB1904AM15 over time.
Figure 2 shows blocking of the binding of human full-length IgE to the FcεRIα receptor protein by anti-IgE antibodies.
Figure 3 shows that blocking of the binding of human full-length IgE to the cell surface CD23 receptor protein by anti-IgE antibodies.
Figures 4A and 4B show blocking of human full-length IgE-induced histamine release from FcεRI/RBL-2H3 by anti-IgE antibodies.
Figure 5 shows histamine release from human whole blood.
Figure 6 shows a curve of the anti-IgE antibodies over time in hFcRn mouse serum.
Figure 7 shows a curve of the anti-IgE antibodies over time in cynomolgus monkey serum.

### Detailed description

Embodiments of the invention herein are described below with specific examples, and those skilled in the art can easily understand other advantages and effects of the invention of the present application from the contents disclosed herein.

### Definition of terms

In this application, the term "IgE" usually refers to antibodies within mammals. IgE is a member of the immunoglobulin family that mediates allergic responses such as asthma, food allerg, type 1 hypersensitivity, and familiar sinusitis suffered on many reasons. IgE is secreted by B cells and expressed on the surface of B cells. B-cells-synthesized IgE is anchored in the B-cell membrane through a transmembrane domain linked to the mature IgE sequence via a short membrane-binding region. IgE can also bind to B cells (as well as monocytes, eosinophils, and platelets) through the attachment of its Fc region to the low-affinity IgE receptor (FcεRII). After mammalian exposure to an allergen, B cells are amplified asexually, which synthesize IgE that binds the allergen. This IgE is respectively released by B cells into the circulation, where it is bound by B cells (via FcεRII) and by mast cells and basophils via so-called high-affinity receptors (FcεRI) found on the surface of mast cells and basophils. Thus, such mast cells and basophils are sensitized to allergens. Next exposure to the allergens will crosslink FcεRI on these cells and thus activate their release of histamine and other factors responsible for clinical hypersensitivity and anaphylaxis.

In this application, the term " antigen-binding protein" usually refers to one or more fragments or portions of an antibody that retain the ability to specifically bind to an antigen (e.g., IgE), or a synthesized variant of antibody fragments that retain the desired binding ability to an antigen. The exhibited antigen-binding function of the antibody can be performed by fragments or some portions of the full-length antibody or variants thereof. Examples include bispecific, dual specific and multispecific formats that can specifically bind to two or more different antigens or several epitopes or discrete epitopes regions of antigens. Examples of antigen-binding proteins may include single chain antibodies (i.e. full length heavy and light chains); Fab, modified Fab, Fab', modified Fab', F(ab')₂, Fv, Fab-Fv, Fab- dsFv, single domain antibody (e.g. VH or VL or VHH) (e.g. as described in WO 2001090190), scFv, bivalent, trivalent or tetravalent antibody, bi-scFv, diabody, tribody, triabody, tetrabody and epitope-antigen binding agents of any of the above (see e.g. Holliger and Hudson, 2005, Nature Biotech. 23(9):1126-1136; Adair and Lawson, 2005, Drug Design Reviews-Online 2(3), 209-217). Methods for producing and making these antibody fragments are well known in the art (see, e.g., Verma et al., 1998, Journal of Immunological Methods, 216, 165-181).

In this application, the term "antibody" generally refers to any immunoglobulin (Ig) molecule comprised of four polypeptide chains (two heavy (H) chains and two light (L) chains) or any functional fragment, mutant, variant or derivative of thereof that retains at least a portion of an epitope binding feature of an Ig molecule to allow it's specific binding to IgE. In full-length antibodies, each heavy chain consists of a heavy chain variable region (abbreviated herein as VH) and a heavy chain constant region (CH). The heavy chain constant region consists of four domains - CH1, hinge, CH2 and CH3 (heavy chains gamma, alpha and delta), or CH1, CH2, CH3 and CH4 (heavy chains mu and epsilon). Each light chain consists of a light chain variable region (abbreviated herein as VL) and a light chain constant region (CL). The light chain constant region consists of one domain, CL. The VH and VL regions can be further subdivided into hypervariable regions, termed complementarity determining regions (CDRs), interspersed with more conserved regions, termed framework regions (FRs). Each VH and VL consists of three CDRs and four FRs arranged from the amino terminus to the carboxy terminus in the following order: FR1, CDR1, FR2, CDR2, FR3, CDR3, FR4. Immunoglobulin molecules can be of any isotype/class (e.g., IgG, IgE, IgM, IgD, IgA, and IgY), class (e.g., IgG1, IgG2, IgG3, IgG4, IgA1, and IgA2), or subclass.

In this application, the term "monoclonal antibody" generally refers to a preparation of antibody molecules that share a common heavy chain amino acid sequence and light chain amino acid sequence, or any functional fragment, mutant, variant or derivative thereof which at least retain the light chain epitope binding characteristics of Ig molecules, in contrast with a "polyclonal" antibody preparations that contain a mixture of different antibodies. Monoclonal antibodies can be produced by several known techniques such as phage, bacterial, yeast or ribosome display as well as classical methods such as hybridoma derived antibodies, for example antibodies secreted by hybridomas by hybridoma technology such as the standard Kohler and Milstein hybridoma method ((1975) Nature 256:495-497).

In this application, the term "omalizumab " generally refers to a recombinant DNA-derived humanized IgG1κ monoclonal antibody that selectively binds to human immunoglobulin E (IgE) under the trade name Xolair^{®}. omalizumab has a molecular weight of approximately 149 kD. It is produced from Chinese hamster ovary cell suspension cultures in medium containing the antibiotic gentamicin (see EP602126 (and SPC/GB06/005 based thereon); WO93/04173; US6267958 (and PTE based thereon)); WO97/04807; WO97/04801; Presta et al. (1993) J. Immunol. 151:2623-2632). When omalizumab is described, it refers to the IgG full-length antibody containing the light chain variable region as shown in SEQ ID NO: 22 and the heavy chain variable region as shown in SEQ ID NO: 50. omalizumab is currently indicated for the treatment of moderate-to-severe persistent asthma in patients with positive skin tests or in vitro responsiveness to chronic aeroallergens and symptoms that are not adequately controlled by inhaled corticosteroids.

In this application, when describing common immunoglobulin portions (CDR-complementarity determining regions, or FR-framework regions) of VH or VL sequences, they are linked in standard order (VH = HFR1.HCDR1.HFR2 HCDR2 HFR3 .HCDR3 .HFR4; VL = LFR1. LCDR1. LFR2. LCDR2. LFR3. LCDR3. LFR4).

In the amino acid numbering of the antibody or antigen-binding protein of the present application, the continuous amino acid sequence derived from the antibody or antigen-binding protein can be numbered according to the Chothia numbering rule. In this application, the terms "Chothia scheme", "Chothia numbering" or "Chothia definition" are used interchangeably and generally refer to a numbering system for amino acid residues (Chothia et al., (1987) J. Mol. Biol., 196, 901-917 (1987)). According to Chothia numbering rules, for omalizumab, Each part of VL (SEQ ID NO: 22) is defined as follows: LFR1 (L1-L23), LCDR1 (L24-L34), LFR2 (L35-L49), LCDR2 (L50-L56), LFR3 (L57-L88), LCDR3 (L89-L97) and LFR4 (L98-L107), each part of VH (SEQ ID NO: 50) is defined as follows: HFR1 (H1-H25), HCDR1 (H26-H32), HFR2 (H33-H51), HCDR2(H52-H56), HFR5 (H57-H94), HCDR3 (H95-H102) and HFR4 (H103-H113), wherein "Lx" or "Hx" (x is an arabic number) represents the amino acid being numbered as x in the light chain (L) variable region or heavy chain (H) variable region, and for VL, L24 represents the amino acid at position 24 of the VL according to Chothia numbering. When inside VL or VH, position Lx or Hx can also be represented by the letter plus a number for the amino acid at position x. For example, for VL of omalizumab, at position L24 is arginine (R), so the amino acid at position 24 of VL may be represented by R24. In this application, the above two designations for position (e.g., L24 and R24) may be used interchangeably. According to the Chothia numbering rules, the amino acid positions of the complementarity-determining regions of omalizumab are shown in Tables 1 to 6 below.

**Table 1. Amino acid numbering of omalizumab LCDR1 (Chothia)**

| Numbering | L24 | L25 | L26 | L27 | L28 | L29 | L30 | L30A | L30B | L30C | L30D | L31 | L32 | L33 | L34 |
|---|---|---|---|---|---|---|---|---|---|---|---|---|---|---|---|
| amino acid | R | A | S | Q | S | V | D | Y | D | G | D | S | Y | M | N |

**Table 2. Amino acid numbering of omalizumab LCDR2 (Chothia)**

| Numbering | L50 | L51 | L52 | L53 | L54 | L55 | L56 |
|---|---|---|---|---|---|---|---|
| amino acid | A | A | S | Y | L | E | S |

**Table 3. Amino acid numbering of omalizumab LCDR3 (Chothia)**

| Numbering | L89 | L90 | L91 | L92 | L93 | L94 | L95 | L96 | L97 |
|---|---|---|---|---|---|---|---|---|---|
| amino acid | Q | Q | S | H | E | D | P | Y | T |

**Table 4. Amino acid numbering of omalizumab HCDR1 (Chothia)**

| Numbering | H26 | H27 | H28 | H29 | H30 | H31 | H31A | H32 |
|---|---|---|---|---|---|---|---|---|
| amino acid | G | Y | S | I | T | S | G | Y |

**Table 5. Amino acid numbering of omalizumab HCDR2 (Chothia)**

| Numbering | H52 | H53 | H54 | H55 | H56 |
|---|---|---|---|---|---|
| amino acid | T | Y | A | G | S |

**Table 6. Amino acid numbering of omalizumab HCDR3 (Chothia)**

| Numbering | H95 | H96 | H97 | H98 | H99 | H100 | H100A | H100B | H100C | H100D | H101 | H102 |
|---|---|---|---|---|---|---|---|---|---|---|---|---|
| amino acid | G | S | H | Y | F | G | H | W | H | F | A | V |

For amino acid mutation of the present application, it is represented by the expression of "amino acid before mutation + position + amino acid after mutation", for example, the amino acid mutation "E55Q" of VL means that the amino acid at position 55 of VL is mutated from glutamic acid (E) to glutamine Amide (Q).

In this application, the terms "Kd", "KD" or "K_{D}" are used interchangeably and generally refer to the dissociation constant for a particular antibody-antigen interaction as known in the art. The strength or affinity of an immunobinding interaction can be expressed in terms of the dissociation constant (kD or kd) of the interaction, where a smaller the KD represents a greater or higher the affinity. Immunobinding properties of selected polypeptides can be quantified using methods well known in the art. One such method involves measuring the rates of antigen binding site/antigen complex formation and dissociation, wherein these rates are dependent on the concentration of complex partners, the affinity of the interaction, and geometric parameters that equally affect the rates in both directions.. Thus, "association rate constants" ("Kon") and "dissociation rate constants" ("Koff") can be determined by calculating the concentrations and actual rates of association and dissociation. The ratio Koff/Kon cancels out all parameters not related to affinity and is equal to the dissociation constant Kd.

The affinity of the antibodies or antigen-binding proteins of the present application and the degree to which they inhibit binding can be determined by one of ordinary skill in the art, using conventional techniques, such as those described by Scatchard et al. (Ann. KY. Acad. Sci. 51:660-672 (1949)), biofilm layer optical interferometry (BLI) techniques using the ForteBio Octet^{®}, or by surface plasmon resonance (SPR) using systems such as Biacore. For the optical interference of the biofilm layer, the bottom end of the inner biosensor is covered by the biofilm layer, which can bind and immobilize biomolecules. When visible light with a certain bandwidth is incident on the biofilm layer vertically, the light is reflected at the two interfaces of the biofilm layer to form an interference wave of a certain wavelength. When the immobilized molecules interact with the molecules in the solution, the thickness of the biofilm layer increases, the interference spectrum curve moves in the direction of increasing wavelength, and the phase shift of the light wave is detected by the workstation in real time. Molecular number changes and associated concentration and kinetic data. For surface plasmon resonance, target molecules are immobilized on a solid phase and exposed to ligands in a mobile phase flowing along a flow cell. If ligand binding to the immobilized target occurs, the local refractive index changes, resulting in a change in the SPR angle, which can be monitored in real time by detecting changes in the intensity of the reflected light. The rate of change of the SPR signal can be analyzed to generate apparent rate constants for the association and dissociation phases of the binding reaction. The ratio of these values yields the apparent equilibrium constant (affinity).

It will be appreciated that the affinity of the antigen-binding proteins provided herein can be altered using any suitable method known in the art. Accordingly, the present application also designs variants of the antigen-binding proteins of the present application, which have improved affinity for IgE. Such variants can be obtained by a number of affinity maturation protocols, including mutating the CDRs (Yang et al., J. Mol. Biol., 254, 392-403, 1995), chain shuffling (Marks et al., Bio/Technology, 10,779- 783, 1992), mutator strains using Escherichia coli (Low et al., J. Mol. Biol., 250, 359-368, 1996), DNA shuffling (Patten et al., Curr. Opin. Biotechnol., 8, 724- 733, 1997), phage display (Thompson et al, J. Mol. Biol., 256, 77-88, 1996) and sexual PCR (Crameri et al, Nature, 391, 288-291, 1998).

In this application, the term "isolated nucleic acid molecule(s)" generally includes DNA molecules and RNA molecules. Nucleic acid molecules can be single-stranded or double-stranded, for example, can be double-stranded DNA. These nucleic acids may be present in whole cells, cell lysates, or in partially purified or substantially pure form. When nucleic acids are isolated and purified from other cellular components or other impurities (such as other cellular nucleic acids or proteins) by standard techniques including alkali/SDS treatment, CsCl banding, column chromatography, agarose gel electrophoresis and other methods known in the art, they are "isolated" or "substantially pure."

In this application, the term "vector" generally refers to a nucleic acid molecule capable of transporting another nucleic acid to which it has been linked. The term "vector(s)" encompasses, but is not limited to, plasmid, viruse, cosmid, and artificial chromosome. One type of vector is a "plasmid," which refers to a circular double-stranded DNA loop into which other DNA segments can be ligated. Another type of vector is a viral vector, in which other DNA segments can be ligated to the viral genome. In general, an engineered vector may contain an origin of replication, multiple cloning site, and a selectable marker. The vector itself is usually a nucleotide sequence, typically a DNA sequence, that contains an insert (transgene) sequence and a larger sequence that serves as the "backbone" or "principal chain" of the vector. Modern vectors may contain additional features in addition to the transgene insert and backbone: promoters, genetic markers, antibiotic resistance, reporter genes, targeting sequences, protein purification tags.

In the present application, the term "cell" generally refers to a cell into which a nucleic acid encoding, for example, a heterologous polypeptide or constituting a shRNA can or may be introduced. Host cells can include prokaryotic cells, which can be used to propagate vectors/plasmids, and eukaryotic cells, which can be used to expression for nucleic acids. For example, eukaryotic cells can be mammalian cells. In another example, the mammalian host cell can be selected from the following mammalian cells: CHO cells (e.g., CHO K1 or CHO DG44), BHK cells, NS0 cells, SP2/0 cells, HEK 293 cells, HEK 293EBNA cells, PER.C6 cells and COS cells. In certain instances, the mammalian cells can be selected from hybridoma, myeloma, and rodent cells. Myeloma cells can include rat myeloma cells (e.g., YB2), and mouse myeloma cells (e.g., NS0, SP2/0). Polypeptides or proteins for pharmaceutical applications can be produced in mammalian cells such as CHO cells, NSO cells, SP2/0 cells, COS cells, HEK cells, BHK cells, cells, and the like.

In this application, the term "pharmaceutically acceptable carrier" generally refers to one or more non-toxic materials that do not interfere with the effectiveness of the biological activity of the active ingredient. Such formulations may conventionally contain salts, buffers, preservatives, compatible carriers, and optionally other therapeutic agents. Such pharmaceutically acceptable carriers may also contain compatible solid or liquid fillers, diluents or encapsulating substances suitable for administration to humans. Other contemplated carriers, excipients, and/or additives that may be used in the formulations described herein include, for example, flavoring agents, antimicrobial agents, sweeteners, antioxidants, antistatic agents, lipids, protein excipients (e.g. serum albumin, gelatin, casein), salt-forming counterions (e.g. sodium), etc. These and other known pharmaceutical carriers, excipients and/or additives suitable for use in the formulations described herein are known in the art, e.g., as described in "Remington's Pharmaceutical Sciences and Practice (Remington: The Science and Practice of Pharmacy, 22nd ed., Pharmaceutical Press (2012) and Physician's Desk Reference Desk Reference)", 66th ed., Medical Economics (2012). The appropriate pharmaceutically acceptable carrier for the desired or required mode of administration, solubility and/or stability can be routinely selected.

In this application, the term "disease associated with abnormal level of IgE" generally refers to any condition or disorder associated with abnormal (e.g., elevated) levels of serum IgE. Exemplary diseases associated with abnormal level of IgE may include, but are not limited to, bronchial asthma, allergic rhinitis, atopic dermatitis, urticaria, allergic responses, or atopic dermatitis.

In this application, the terms "polypeptide", "peptide", "protein" and "proteins" can be used interchangeably and generally refer to polymers of amino acids of any length. The polymer may be linear or branched, it may contain modified amino acids, and it may be interrupted by non-amino acids. These terms also encompass amino acid polymers that have been modified. These modifications may include: disulfide bond formation, glycosylation, lipidation, acetylation, phosphorylation, or any other manipulation (e.g., binding to labeling components). The term "amino acid" includes natural and/or non-natural or synthetic amino acids, including glycine and D and L optical isomers, as well as amino acid analogues and peptide mimics.

In this application, the terms "polynucleotide", "nucleotide", "nucleotide sequence", "nucleic acid" and "oligonucleotide" can be used interchangeably and generally refer to polymeric forms of nucleotides with any length, such as deoxyribonucleotides or ribonucleotides, or analogues thereof. A polynucleotide can have any three-dimensional structure and can perform any function, known or unknown. The following are non-limiting examples of polynucleotides: coding or non-coding regions of genes or gene fragments, multiple loci (one locus) defined by ligation analysis, exons, introns, messenger RNA (mRNA), transfer RNA, ribosomal RNA, short interfering RNA (siRNA), short hairpin RNA (shRNA), micro-RNA (miRNA), ribozyme, cDNA, recombinant polynucleotide, branched polynucleotide, plasmid, vector, isolated DNA of any sequence, isolated RNA of any sequence, nucleic acid probes, and primers. A polynucleotide may contain one or more modified nucleotides, such as methylated nucleotides and nucleotide analogues. If present, modification of the nucleotide structure can be performed before or after polymer assembly. The sequence of nucleotides can be interrupted by non-nucleotide components. Polynucleotides can be further modified after polymerization, such as by conjugation to labeled components.

In addition to the specific proteins and nucleotides mentioned herein, the present application may also include functional variants, derivatives, analogues, homologues, and fragments thereof.

The term "functional variant" refers to a polypeptide having substantially the same amino acid sequence as the naturally occurring sequence or encoded by substantially the same nucleotide sequence and capable of possessing one or more activities of the naturally occurring sequence. In the context of this application, a variant of any given sequence refers to sequence in which a particular sequence of residues (whether amino acid or nucleotide residues) has been modified such that the polypeptide or polynucleotide substantially retains at least one endogenous functions. Variant sequences can be obtained by addition, deletion, substitution, modification, replacement and/or variation of at least one amino acid residues and/or nucleotide residues presenting in a naturally occurring protein and/or polynucleotide, so long as the the original functional activity remains.

In the present application, the term "derivative" generally refers to polypeptide or polynucleotide including any substitution, variation, modification, replacement, deletion and /or addition of one (or more) amino acid residue of If or the polypeptide or polynucleotide of the present application, so long as the resulting polypeptide or polynucleotide substantially retains at least one of its endogenous functions.

In this application, the term "analogue" generally refers to a polypeptide or polynucleotide including any mimetic of the polypeptide or polynucleotide, i.e. chemical compounds possessing at least one endogenous functions of the polypeptide or polynucleotide that the mimetic mimics.

Generally, amino acid substitutions, such as at least 1 (e.g., 1, 2, 3, 4, 5, 6, 7, 8, 9, 10, or 20 more) amino acid substitutions, can be made, so long as the modified sequence remains substantially desired activity or ability. Amino acid substitutions can include the use of non-naturally occurring analogues.

The proteins or polypeptides used in the present application may also have deletions, insertions or substitutions of amino acid residues that produce silent changes and result in functionally equivalent proteins. Deliberate amino acid substitutions can be made based on similarity in polarity, charge, solubility, hydrophobicity, hydrophilicity, and/or amphiphilic nature with the residues, so long as endogenous function remains. For example, negatively charged amino acids include aspartic acid and glutamic acid; positively charged amino acids include lysine and arginine; and amino acids containing uncharged polar headgroups with similar hydrophilicity values include asparagine, glutamine, serine, threonine and tyrosine.

In this application, the term "homologue" generally refers to an amino acid sequence or nucleotide sequence that has certain homology to a wild-type amino acid sequence and a wild-type nucleotide sequence. The term "homology" may be equivalent to sequence "identity". Homologous sequences can include amino acid sequences at least 80%, 85%, 90%, 99.1 %, 99.2%, 99.3%, 99.4%, 99.5%, 99.6%, 99.7%, 99.8%, or 99.9% identical to the subject sequence. Typically, a homologue will contain the same active site, etc., as the subject amino acid sequence. Homology can be considered in terms of similarity (i.e. amino acid residues with similar chemical properties/functions), or it can be expressed in terms of sequence identity. In the present application, the sequence having a percent identity to any one of the SEQ ID NO of an amino acid sequence or a nucleotide sequence means the sequence having said percent identity over the entire length of the referenced SEQ ID NO.

To determine sequence identity, sequence alignment can be performed, which can be performed by various means known to those skilled in the art, e.g., using BLAST, BLAST-2, ALIGN, NEEDLE, or Megalign (DNASTAR) software, and the like. Those skilled in the art can determine appropriate parameters for alignment, including any algorithms needed to achieve optimal alignment among the full-length sequences being compared.

In this application, the term "and/or" should be understood to mean either or both of the alternatives.

In this application, the term "comprising" generally means including the expressly specified features, but not excluding other elements.

In this application, the term "about" generally refers to a change within a range of 0.5%-10% above or below the specified value, such as a change within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

### Embodiments

In one aspect, the present application provides an antigen-binding protein, the antigen-binding protein may comprise an antibody light chain variable region (VL), wherein compared to the light chain variable region of omalizumab, (i.e. amino acid sequence as shown in SEQ ID NO: 22), the VL of the antigen-binding protein described herein may comprise amino acid mutations at one or two positions selected from the group consisting of E55 and V104.

In the present application, the VL of the antigen-binding protein may comprise amino acid mutations at one or more (e.g., 1, 2, 3, 4, or 5) positions selected from the group consisting of: D30b, V29, D30, Y30a and G30c.

For example, the VL of the antigen-binding protein may comprise amino acid mutations at positions of the following group: D30b, E55 and V104.

For example, the VL may comprise amino acid mutations at positions D30b, E55, V104, V29, D30 and Y30a.

For example, the VL may comprise amino acid mutations at positions D30b, E55, V104, D30, Y30a and G30c.

For example, the VL may comprise amino acid mutations at positions D30b, E55, V104 and Y30a.

For example, the VL may comprise amino acid mutations at positions D30b, E55, V104, Y30a and G30c.

For example, the VL may comprise amino acid mutations at positions D30b, E55, V104, D30, Y30a and G30c.

For example, the VL may comprise amino acid mutations at positions D30b, E55, V104, D30 and Y30a.

In the present application, the amino acid mutation at the position E55 may include E55Q.

In the present application, the amino acid mutation at the position V104 may include V104L.

In the present application, the amino acid mutation at the position D30b may include D30bE.

In the present application, the amino acid mutation at the position V29 may include V29L.

In the present application, the amino acid mutation at the position Y30a may include any one selected from the group consisting of Y30A, Y30aS and Y30aD.

In the present application, the amino acid mutation at the position D30 includes any one selected from the group consisting of D30Y, D30A, D30E, D30F, D30G and D30N.

In the present application, the amino acid mutation at the position G30c may include any one selected from the group consisting of G30cA, G30cY and G30cW.

In the present application, the VL may comprise the following group of amino acid mutations: D30bE, E55Q and V104L.

In the present application, the VL may comprise amino acid sequence as shown in SEQ ID NO: 57.

In the present application, the VL of the antigen-binding protein may comprise amino acid sequence as shown in SEQ ID NOs: 23-34.

For example, the VL of the antigen-binding protein may comprise amino acid mutations D30bE, E55Q, V104L, V29L, D30Y and Y30aD.

For example, the VL may comprise amino acid mutations D30bE, E55Q, V104L, D30A, Y30aD and G30cW.

For example, the VL may comprise amino acid mutations 30bE, E55Q, V104L and Y30aA.

For example, the VL may comprise amino acid mutations D30bE, E55Q, V104L and Y30aD.

For example, the VL may comprise amino acid mutations D30bE, E55Q, V104L and Y30aS.

For example, the VL may comprise amino acid mutations D30bE, E55Q, V104L, Y30aD and G30cY

For example, the VL may comprise amino acid mutations D30bE, E55Q, V104L, D30E, Y30aD and G30cW.

For example, the VL may comprise amino acid mutations D30bE, E55Q, V104L, D30G, Y30aD and G30cA.

For example, the VL may comprise amino acid mutations D30bE, E55Q, V104L, D30N, Y30aD and G30cA.

For example, the VL may comprise amino acid mutations D30bE, E55Q, V104L, D30N, Y30aD and G30cW.

For example, the VL may comprise amino acid mutations D30bE, E55Q, V104L, D30F and Y30aD.

In the present application, the antigen-binding protein may further comprise a light chain constant region. For example, the light chain constant region can be from a human kappa light chain. For example, the light chain constant region may comprise amino acid sequence as shown in SEQ ID NO: 59.

In the present application, the antigen-binding protein may also include antibody light chains. The antibody light chain may include the antibody light chain variable region and the antibody light chain constant region. For example, the antibody light chain of the antigen-binding protein described herein may comprise any one of amino acid sequence as shown in SEQ ID NOs: 23-34.

The antigen-binding protein described in the present application may comprise an antibody heavy chain variable region (VH), wherein compared with the heavy chain variable region of omalizumab (the amino acid sequence as shown in SEQ ID NO: 50), the VH of the antigen-binding protein can comprise amino acid mutations at one or more (e.g., 1, 2, 3 or 4) positions selected from the group consisting of I37, A49, S50 and D54.

In the present application, the VH of the antigen-binding protein may further comprise amino acid mutations selected from the group consisting of at one or more (e.g., 1, 2, 3, 4, 5, 6 or 7) positions: N60, P61, 167, T30, S31, S96 and H97.

For example, the VH of the antigen-binding protein may comprise amino acid mutations at positions of the following group: N60, P61, 167, 137, A49, S50 and D54.

For example, the VH may comprise amino acid mutations at positions N60, P61, I67, I37, A49, S50, D54, S96 and H97.

For example, the VH may comprise amino acid mutations at positions N60, P61, I67, I37, A49, S50, D54 and T30.

For example, the VH may comprise amino acid mutations at positions N60, P61, I67, I37, A49, S50, D54 and S96.

For example, the VH may comprise amino acid mutations at positions N60, P61, I67, I37, A49, S50, D54 and S31.

For example, the VH may comprise amino acid mutations at positions N60, P61, I67, I37, A49, S50, D54 and H97.

In the present application, the amino acid mutation at position 137 may include 137V.

In the present application, the amino acid mutation at position A49 may include A49S.

In the present application, the amino acid mutation at position S50 may include S50V

In the present application, the amino acid mutation at position D54 may include D54A.

In the present application, the amino acid mutation at position N60 may include N60A.

In the present application, the amino acid mutation at position P61 may include P61D.

In the present application, the amino acid mutation at position I67 may include I67F.

In the present application, the amino acid mutation at position T30 may include T30R.

In the present application, the amino acid mutation at position S31 may include S31Q.

In the present application, the amino acid mutation at position S96 may include S96T.

In the present application, the amino acid mutation at position H97 may include H97N.

In the present application, the VH may comprise the following group of amino acid mutations: N60A, P61D, I67F, I37V, A49S, S50V and D54A.

In the present application, the VH may comprise amino acid sequence as shown in SEQ ID NO: 58.

In the present application, the VH of the antigen-binding protein may comprise any one of amino acid sequence as shown in SEQ ID NOs: 51-56.

For example, the VH of the antigen-binding protein may comprise amino acid mutations N60A, P61D, I67F, 137V, A49S, S50V, D54A, S96T and H97N.

For example, the VH may comprise amino acid mutations N60A, P61D, I67F, 137V, A49S, S50V, D54A and T30R.

For example, the VH may comprise amino acid mutations N60A, P61D, I67F, 137V, A49S, S50V, D54A and S96T.

For example, the VH may comprise amino acid mutations N60A, P61D, I67F, 137V, A49S, S50V, D54A and S3IQ.

For example, the VH may comprise amino acid mutations N60A, P61D, I67F, 137V, A49S, S50V, D54A and H97N.

In the present application, the antigen-binding protein may comprise HCDR1, HCDR2 and HCDR3, and the HCDR1 may comprise the amino acid sequence as shown in SEQ ID NO: 62, the HCDR2 may comprise amino acid sequence as shown in SEQ ID NO: 42, and the HCDR3 may comprise amino acid sequence as shown in SEQ ID NO: 63.

For example, the antigen-binding protein can comprise HCDR1, HCDR2 and HCDR3, and the HCDR1 can comprise any one of amino acid sequence as shown in SEQ ID NO: 36-37, the HCDR2 may comprise amino acid sequence as shown in SEQ ID NO: 42, and the HCDR3 may comprise any one of amino acid sequence as shown in SEQ ID NO: 45-48.

In the present application, the antigen-binding protein may comprise LCDR1, LCDR2 and LCDR3, and the LCDR1 may comprise amino acid sequence as shown in SEQ ID NO: 64, the LCDR2 may comprise amino acid sequence as shown in SEQ ID NO: 17, and the LCDR3 may comprise amino acid sequence as shown in SEQ ID NO: 19.

For example, the antigen-binding protein can comprise LCDR1, LCDR2 and LCDR3, and the LCDR1 can comprise any one of amino acid sequence as shown in SEQ ID NO: 3-14, the LCDR2 may comprise amino acid sequence as shown in SEQ ID NO: 17, and the LCDR3 may comprise amino acid sequence as shown in SEQ ID NO: 19.

In the present application, the antigen-binding protein may comprise HCDR1, HCDR2, HCDR3, LCDR1, LCDR2 and LCDR3, and the HCDR1, HCDR2, HCDR3, LCDR1, LCDR2 and LCDR3 may respectively comprise amino acid sequences as shown in SEQ ID NO: 62, 42, 63, 64, 17 and 19.

For example, the antigen-binding protein may comprise HCDR1, HCDR2, HCDR3, LCDR1, LCDR2 and LCDR3, and the HCDR1 may comprise any one of amino acid sequence as shown in SEQ ID NO: 36-37, the HCDR2 may comprise amino acid sequence as shown in SEQ ID NO: 42, the HCDR3 may comprise any one of amino acid sequence as shown in SEQ ID NO: 45-48, the LCDR1 may comprise any one of amino acid sequence as shown in SEQ ID NO: 3-14, the LCDR2 may comprise amino acid sequence as shown in SEQ ID NO: 17, and the LCDR3 may comprise amino acid sequence as shown in SEQ ID NO: 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 45, 3, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 46, 4, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 37, 42, 45, 5, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 47, 6, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 45, 7, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 45, 8, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 48, 9, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 45, 10, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 48, 11, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 45, 12, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 47, 12, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 36, 42, 47, 13, 17 and 19.

For example, the antigen-binding protein may comprise HCDR1, HCDR2, HCDR3, LCDR1, LCDR2 and LCDR3, which may respectively comprise the amino acid sequences as shown in SEQ ID NO: 38, 42, 45, 14, 17 and 19.
the present application, the antigen-binding protein may comprise framework regions HFR1, HFR2, HFR3, HFR4, LFR1, LFR2, LFR3 and LFR4, which may respectively comprise SEQ ID NO: amino acid sequences as shown in 35, 40, 44, 49, 1, 15, 18 and 21.

In the present application, the antigen-binding protein may further comprise an antibody heavy chain constant region. In certain instances, the antibody heavy chain constant region can be derived from a human IgG constant region. For example, the human IgG constant region may comprise a human IgG1 constant region. In certain instances, the human IgG1 constant region may comprise one or more amino acid mutations selected from the group consisting of M252Y, S254T, and T256E (Chothia numbering rules). For example, the human IgG1 constant region may comprise the following group of amino acid mutations: M252Y, S254T and T256E (Chothia numbering rules). In certain instances, the antibody heavy chain constant region of the antigen-binding protein described herein may comprise the any one of amino acid sequence as shown in SEQ ID NOs: 60-61.

In the present application, the antigen-binding protein may also include an antibody heavy chain. The antibody heavy chain may include the antibody heavy chain variable region and the antibody heavy chain constant region.

In the present application, the antigen-binding protein may comprise an antibody light chain variable region (VL) and an antibody heavy chain constant region (VH), wherein, compared with the amino acid sequence as shown in SEQ ID NO: 22, the VL may comprise amino acid mutations at one or two positions selected from the group consisting of E55 and V104, and compared with the amino acid sequence as shown in SEQ ID NO: 50, the VH may comprise amino acid mutations at one or more positions (e.g., 1, 2, 3, 4, or 5) selected from the group consisting of: I37, A49, S50, and D54.

In certain instances, the VL of the antigen-binding protein can comprise amino acid mutations at positions of the following group: D30b, E55 and V104, and the VH can comprise amino acid mutations at positions of the following group: N60, P61, 167, 137, A49, S50 and D54.

For example, the VL of the antigen-binding protein may comprise amino acid mutations at the following group of positions D30b, E55, V104, V29, D30 and Y30a, and the VH may comprise amino acid mutations at the following group of positions N60, P61, I67, I37, A49, S50, D54, S96 and H97.

For example, the VL of the antigen-binding protein of the present application may comprise the amino acid mutations at positions of the following group: D30b, E55, V104, D30, Y30a and G30c, and the VH may comprise the amino acid mutations at positions of the following group: N60, P61, I67, I37, A49, S50, D54 and T30 .

For example, the VL can comprise the amino acid mutations at the following group of positions D30b, E55, V104 and Y30a, and the VH can comprise the amino acid mutations at the following group of positions N60, P61, 167, I37, A49, S50, D54 and S96.

For example, the VL may comprise the amino acid mutations at positions of the following group: D30b, E55, V104 and Y30a, and the VH may comprise the amino acid mutations at positions of the following group: N60, P61, 167, 137, A49, S50 and D54.

For example, the VL can comprise the amino acid mutations at positions of the following group: D30b, E55, V104, Y30a and G30c, and the VH can comprise the amino acid mutations at positions of the following group: N60, P61, 167, I37, A49, S50 and D54.

For example, the VL can comprise the amino acid mutations at positions of the following group: D30b, E55, V104 and Y30a, and the VH can comprise the amino acid mutations at positions of the following group: N60, P61, 167, I37, A49, S50, D54 and H97.

For example, the VL can comprise the amino acid mutations at positions of the following group: D30b, E55, V104, D30, Y30a and G30c, and the VH can comprise the amino acid mutations at positions of the following group: N60, P61, I67, I37, A49, S50 and D54.

For example, the VL may comprise the amino acid mutations at positions of the following group: D30b, E55, V104, D30 and Y30a, and the VH may comprise the amino acid mutations at positions of the following group: N60, P61, 167, 137, A49, S50, D54 and H97.

For example, the VL can comprise the amino acid mutations at positions of the following group: D30b, E55, V104, D30, Y30a and G30c, and the VH can comprise the amino acid mutations at positions of the following group: N60, P61, 167, 137, A49, S50, D54 and S96.

For example, the VL can comprise the amino acid mutations at positions of the following group: D30b, E55, V104, D30, Y30a and G30c, and the VH can comprise the amino acid mutations at positions of the following group: N60, P61, 167, 137, A49, S50, D54 and S31.

In the present application, the species of amino acid mutations at the respective positions may be those mentioned above.

In the present application, the VL of the antigen-binding protein may comprise any one of amino acid sequence as shown in SEQ ID NOs: 23-34, and the VH may comprise as any one of amino acid sequence as shown in SEQ ID NOs: 51-56.

For example, the VL may comprise amino acid sequence as shown in SEQ ID NO: 23, and the VH may comprise amino acid sequence as shown in SEQ ID NO: 51.

For example, the VL may comprise amino acid sequence as shown in SEQ ID NO: 24, and the VH may comprise amino acid sequence as shown in SEQ ID NO: 52.

For example, the VL may comprise amino acid sequence as shown in SEQ ID NO: 25, and the VH may comprise amino acid sequence as shown in SEQ ID NO: 53.

For example, the VL may comprise amino acid sequence as shown in SEQ ID NO: 26, and the VH may comprise amino acid sequence as shown in SEQ ID NO: 54.

For example, the VL may comprise amino acid sequence as shown in SEQ ID NO: 27, and the VH may comprise amino acid sequence as shown in SEQ ID NO: 51.

For example, the VL may comprise amino acid sequence as shown in SEQ ID NO: 28, and the VH may comprise amino acid sequence as shown in SEQ ID NO: 51.

For example, the VL may comprise amino acid sequence as shown in SEQ ID NO: 29, and the VH may comprise amino acid sequence as shown in SEQ ID NO: 55.

For example, the VL may comprise amino acid sequence as shown in SEQ ID NO: 30, and the VH may comprise amino acid sequence as shown in SEQ ID NO: 51.

For example, the VL may comprise amino acid sequence as shown in SEQ ID NO: 31, and the VH may comprise amino acid sequence as shown in SEQ ID NO: 55.

For example, the VL may comprise amino acid sequence as shown in SEQ ID NO: 32, and the VH may comprise amino acid sequence as shown in SEQ ID NO: 51.

For example, the VL may comprise amino acid sequence as shown in SEQ ID NO: 32, and the VH may comprise amino acid sequence as shown in SEQ ID NO: 54.

For example, the VL may comprise amino acid sequence as shown in SEQ ID NO: 33, and the VH may comprise amino acid sequence as shown in SEQ ID NO: 54.

For example, the VL may comprise amino acid sequence as shown in SEQ ID NO: 34, and the VH may comprise amino acid sequence as shown in SEQ ID NO: 56.

The antigen-binding proteins described in the present application have high chemical stability. For example, it is stable at high temperature, low pH and/or repeated freezing and thawing. For example, at 40°C for 0 to 28 days, or at pH 5.0 and 40°C for 0-28 days, the antigen-binding protein of the present application produces less aggregates by at least 5% (e.g., at least 10%, 15%, 20%, 25%, 30% or higher) compared to omalizumab (e.g., detected by Size Exclusion High Performance Liquid Chromatography SEC-HPLC). For example, at 40°C for 0 to 28 days, or at pH 5.0 and 40°Cfor 0-28 days, the main peak content of the antigen-binding protein described herein is at least 5% (e.g., at least 10%, 15%, 20% or higher) higher than that of omalizumab (e.g., by whole-column imaging capillary isoelectric focusing electrophoresis (iCIEF detection).

The antigen-binding proteins described herein have high binding affinity to human IgE or fragments thereof, for example, with a *K_{D}* value less than 5 × 10⁻¹⁰M (e.g., less than 4 × 10⁻¹⁰M, 3 × 10⁻¹⁰M, 2 × 10⁻¹⁰M, 2.8 × 10⁻¹⁰M, 2.6 × 10⁻¹⁰M, 2.4 × 10⁻¹⁰M, 2.2 × 10⁻¹⁰M, 2.0 × 10⁻¹⁰M, 1.8 × 10⁻¹⁰M, 1.6 × 10⁻¹⁰M, 1.410⁻¹⁰M, 1.2 × 10⁻¹⁰M, 1.0 × 10⁻¹⁰ M or even less) for binding to human IgE CH3-CH4 (e.g., as detected by Octet Red); for another example, with a *K_{D}* value less than 2 × 10⁻⁹ M (e.g., less than 1.5 × 10⁻⁹ M, 1.2 × 10⁻⁹ M, 1 × 10⁻⁹M or even less) for binding to human full-length IgE (e.g., as detected by Octet Red); for more another example, with a *K_{D}* value less than 5 × 10⁻⁹ M (e.g., less than 4.8 × 10⁻⁹ M, 4.6 × 10⁻⁹ M, 4.4 × 10⁻⁹ M, 4.2 × 10⁻⁹ M, 4.0 × 10⁻⁹ M, 3.8 × 10⁻⁹ M or even less) for binding to human full-length IgE (e.g., as detected by Biacore).

The antigen binding protein described in this application can bind to monkey IgE with high affinity, facilitating pharmacodynamic studies in preclinical animal models, for example with a *K_{D}* value less than 3.0X 10⁻¹⁰M (e.g., less than 2.5 × 10⁻¹⁰M, 2.4 × 10⁻¹⁰M, 2.3 × 10⁻¹⁰M, 2.2 × 10⁻¹⁰M, 2.1 × 10⁻¹⁰M, 2.0 × 10⁻¹⁰M, 1.9 × 10⁻¹⁰M, 1.8 × 10⁻¹⁰ M or even less) for binding to IgE CH3-CH4 of cynomolgus monkeys (e.g., as detected by Octet Red), for another example, with a *K_{D}* value less than 4.0 × 10-9 M (e.g., less than 3.9 × 10⁻⁹ M, 3.8 × 10⁻⁹ M, 3.7 × 10⁻⁹ M, 3.6 × 10⁻⁹ M, 3.5 × 10⁻⁹ M or even less) for binding monkey IgE CH3-CH4 (e.g., as detected by Biacore).

The antigen-binding proteins described in this application can effectively block the binding of IgE to the IgE Fc receptor (FcεRI), for example, with EC₅₀ values less than 1.5 nM (e.g., less than 1.4 nM, 1.3 nM, 1.2 nM, 1.1 nM, 1.0 nM, 0.9 nM or even less) for blocking binding of human full-length IgE to FcεRI (e.g., as detected by ELISA), for another example, with EC₅₀ values less than 4.0 nM (e.g., less than 3.9 nM, 3.8 nM, 3.7 nM, 3.6 nM, 3.5nM, 3.4nM, 3.3nM, 3.2nM, 3.1nM, 3.0nM or even less) for blocking IgE -stimulated histamine release from FcεRI -expressing cells.

The antigen-binding protein of the present application has a long half-life in blood, which can reduce the frequency of its injection and facilitate the control of the patient's disease. For example, the half-life of the antigen-binding proteins described herein is increased by 10%, 20%, 30%, 40%, 50%, 60%, 70%, 80%, 90%, 100% or more than omalizumab, as calculated by non-compartmental model analysis or free IgE concentration using an ELISA assay.

### Nucleic acid molecules, vectors and cells

The present application also provides an isolated nucleic acid molecule encoding the heavy and/or light chain of the antigen-binding protein of the present application. Suitably, the nucleic acid molecule encodes a heavy or light chain of an antigen-binding protein of the present application. The nucleic acid molecules of the present application may comprise synthetic DNA, e.g., produced by chemical processing, cDNA, genomic DNA, or any combination thereof.

Nucleic acid molecules encoding the antigen-binding proteins of the present application can be obtained by methods well known to those skilled in the art. For example, nucleic acid molecules encoding part or all of antibody heavy and light chains can be synthesized as desired from the assayed nucleic acid molecules or based on the corresponding amino acid sequences. Nucleic acid molecules encoding the antigen-binding proteins of the present application can be prepared using standard techniques of molecular biology. Desired nucleic acid molecules can be synthesized in completely or in part using oligonucleotide synthesis techniques. Site-directed mutagenesis and polymerase chain reaction (PCR) techniques can be used when appropriate.

The present application also relates to a cloning or expression vector comprising one or more nucleic acid molecules of the present application. Accordingly, there is provided a cloning or expression vector comprising one or more nucleic acid molecules encoding the antigen-binding proteins of the present application. Alternatively, the cloning or expression vector may comprise two nucleic acid molecules encoding the light and heavy chains of the antigen-binding proteins of the present application, respectively, and a suitable signal sequence.

The present application also provides a host cell comprising one or more cloning or expression vectors comprising one or more nucleic acid molecules encoding the antigen-binding proteins of the present application. Any suitable host cell/vector system can be used to express nucleic acid molecules encoding the antigen-binding proteins of the present application. Bacteria such as E. coli and other microbial systems can be used, or eukaryotic such as mammalian, host cell expression systems can also be used. Suitable mammalian host cells include CHO, myeloma or hybridoma cells.

### Methods for preparation

The present application also provides a method for producing the antigen-binding protein described herein, comprising culturing the cell under conditions allowing expression of the antigen-binding protein. The antigen-binding protein may comprise only heavy or light chain polypeptides, in which case only the coding sequence for the heavy or light chain may be transfected into the host cell. To produce a product comprising both heavy and light chains, cell lines can be transfected with two vectors, a first vector encoding the light chain polypeptide and a second vector encoding the heavy chain polypeptide. Alternatively, a single vector can be used, and the vector includes sequences encoding light and heavy chain polypeptides.

The present application provides a method for culturing host cells, and expressing, isolating and purifying the antigen-binding protein or fragments thereof.

In one embodiment, provided is a method for purifying an antigen-binding protein comprising the steps of: performing anion exchange chromatography in a non-binding mode such that impurities are retained on the column and antibodies are eluted.

For one example, the purification can employ affinity capture on a protein A column followed by titration. For one example, the purification can employ affinity capture on a Protein G column followed by HPLC titration. For one example, the purification can employ affinity capture on an IgE column followed by titration. The method for purifying may also include one or more filtration steps, such as diafiltration steps or HPLC filtration steps.

### Pharmaceutical compositions, methods and uses

Because the antigen-binding protein of the present application is suitable for the treatment and/or prevention of pathological conditions, the present application also provides a pharmaceutical or diagnostic composition comprising the antigen-binding protein of the present application in combination with one or more pharmaceutical acceptable excipient, diluent or carrier. Therefore, the present application also provides a use of the antigen-binding protein of the present application in preparation of the pharmaceutical compositions.

The application also provides a method for preparing a pharmaceutical or diagnostic composition comprising adding and mixing the antibody or antigen binding agent of the application together with one or more pharmaceutically acceptable excipients, diluents or carriers. The antigen-binding protein can serve as the sole active ingredient in a pharmaceutical or diagnostic composition, or it can be accompanied by other active ingredients including other antibody or non-antibody ingredients, such as steroids or other drug molecules, especially drug molecules whose half-life is not related to IgE binding.

The composition may be administered to a patient individually or may be administered in combination with (e.g. simultaneously, sequentially or separately) other agents, drugs or hormones.

On the other hand, the present application provides a use of the antigen-binding protein, the nucleic acid molecule, the vector, the cell and/or the composition in prepartion of a medicament for alleviating or treating a disease associated with abnormal level of IgE.

In another aspect, the application provides a method of alleviating or treating a disease associated with abnormal level of IgE, wherein the method comprises administering the antigen-binding protein, the nucleic acid molecule, the vector, the cell and/or the composition to a subject in need thereof.

The pharmaceutical composition may comprise a therapeutically effective amount of an antibody or antigen-binding protein of the present application. As used herein, the term "therapeutically effective amount" refers to an amount of a therapeutic agent required to treat, ameliorate or prevent a targeted disease or condition or to exhibit a detectable therapeutic or preventativ effect. For any disclosed antibody or antigen-binding protein, a therapeutically effective amount can be estimated initially in cell culture assays or in animal models, typically in rodents, rabbits, dogs, pigs, or primates. Animal models can also be used to determine appropriate concentration ranges and routes of administration. This information can then be used to determine dosages and routes suitable for administration to humans. The precise therapeutically effective amount for a human subject will depend on the severity of the disease condition, the general health of the subject, the age, weight and sex of the subject, diet, time and frequency of administration, drug combination, sensitivity of response and tolerance/response to therapy.

The pharmaceutical compositions of the present application can be administered by a variety of routes including, but not limited to, oral, intravenous, intramuscular, intraarterial, intramedullary, intrathecal, intraventricular, transcutaneous, transdermal, subcutaneous, intraperitoneal, intranasal, enteral, topical, sublingual, intravaginal or rectal route. The pharmaceutical compositions of the present invention can also be administered using needle-free injection device. Typically, therapeutic compositions are prepared as injectables as liquid solutions or suspensions. Solid forms suitable for solution in, or suspension in, liquid vehicles prior to injection can also be prepared.

A therapeutic dose of the pharmaceutical composition comprising the antigen-binding protein of the present application does not exhibit significant toxicological effects in vivo. Also, by administering successive doses of the antigen-binding proteins of the present application, the level of IgE activity in a subject can be maintained at an appropriately normal (e.g., reduced) level.

Not to be limited by any theory, the following examples are only intended to illustrate the fusion protein, preparation method and use, or the like of the present application, and are not intended to limit the scope of the invention of the present application.

### Example

### Example 1 Design and expression of omalizumab engineered optimized antibodies

Based on the light and heavy chain variable regions of omalizumab (the light chain variable region sequence is shown in SEQ ID NO: 22, the heavy chain variable region sequence is shown in SEQ ID NO: 50), optimized design, humanized mutation of light and heavy chains and removal of post-translational modification (PTM) site are carried out to obtain new light and heavy chains variable regions.

Then, codon optimization of the protein sequences of the light and heavy chains of the full-length recombinant antibody were performed, and the codon-optimized DNA fragments (Genscript) were genetically synthesized. After cloning, the synthesized gene fragments with the form of IgG1 were interted into the expression vector pcDNA3.4 (Life Technologies). After the expression plasmid was amplified and the plasmid was extracted, the two plasmids were co-transfected into ExpiCHO cells (ThermoFisher Scientific, A29133), and then the antibody was transiently expressed according to the method of the manufacturer's ExpiCHO expression system by incubating Expi CHO cells to a density of 6 × 10⁶ /mL in a total culture volume of 25 ml of medium at 8% carbon dioxide concentration, 36.5°C, and using ExpiFectamine transfection reagent to transform each 10 µg of antibody light chain and heavy chain expression plasmids into cells, respectively; one day after transfection, adding 150 µL, and 4 mL of ExpiCHO enhancer and ExpiCHO supplementary material to the cultured cells, and culturing the cells for 9 days, followd by centrifugation at 3500 rpm at 4 degrees to obtain the supernatant. AmMagTM Protein A magnetic beads (Genscript, L00695) and antibody expression supernatant were mixed, followd by incubating at room temperature for 2 hours, and washing twice with PBS before discarding the supernatant, and then adding an appropriate amount of elution buffer Protein G or A SefinoseTM Elution buffer (Sangon, C600481), followed by fully mixing before placing it on a tube rack for an incubation of 5 minutes, during incunation period the magnetic beads were resuspend for 2-3 times followed by eluting 2 times, before immediately adding an appropriate amount of neutralizing solution 1M Tris-HCl, pH7.5 (Sangon, B548124) for neutralization and storage. The purified antibody obtained is shown in Table 7 (AB1904HzL0H0 represents for omalizumab).

**Table 7 Antibody after humanization and PTM mutation removal**

| Antibody number | light chain variable region mutation | heavy chain variable region mutation |
|---|---|---|
| AB1904HzL0H0 | none | none |
| AB1904L1p1H3 p2 | D30bE, E55Q, V104L | I37V,A49S,S50V,D54A,N60A,P61D,I67F |
| AB1904L13p1H 3p3 | D30bE,E55Q,D94Y,V 104L | I37V,A49S,S50V,D54G,N60A,P61D,I67F |
| AB1904L2p1H4 p2 | D30bE, E93N, V104L | I37V,A49S,S50V,D54A,N60A,P61D,I67F, D73N,F78L |
| AB1904L2p1H4 p3 | D30bE, E93N, V104L | I37V,A49S,S50V,D54G,N60A,P61D,I67F, D73N,F78L |
| AB1904L1p1H5 p2 | D30bE, E55Q, V104L | I37V,A49S,S50V,D54A,N60A,I67F,F78L |
| AB1904L2p1H5 p2 | D30bE, E93N, V104L | I37V,A49S,S50V,D54A,N60A,I67F,F78L |
| AB1904L12p1H 5p2 | D30bE, E55Q, E93N, V104L | I37V,A49S,S50V,D54A,N60A,I67F,F78L |
| AB1904L13p1H 5p2 | D30bE,E55Q,D94Y,V104L | I37V,A49S,S50V,D54A,N60A,I67F,F78L |
| AB1904L1p1H5 p3 | D30bE, E55Q, V104L | I37V,A49S,S50V,D54G,N60A,I67F,F78L |
| AB1904L2p1H5 p3 | D30bE, E93N, V104L | I37V,A49S,S50V,D54G,N60A,I67F,F78L |
| AB1904L12p1H 5p3 | D30bE, E55Q, E93N, V104L | I37V,A49S,S50V,D54G,N60A,I67F,F78L |
| AB1904L13p1H 5p3 | D30bE,E55Q,D94Y,V104L | I37V,A49S,S50V,D54G,N60A,I67F,F78L |

### Example 2 Assay for Human IgE Affinity

Antibodies were first tested for affinity with biotinylated human IgE CH3-CH4 (Biotin-hIgE CH3-CH4, Sino Biological, Cat. No. 29702-H08H, biotin-labeled in-house) and sorted by off rate with the following experimental proceses.
(1) Dilution of antigen and antibody: Both antigen and antibody were diluted with 1×Kinetic Buffer (Kinetic Buffer, Fortebio, 18-1105), and the concentration of antigen for use was 1 µg/ml, and the concentration of antibody for use was 100 nM.
(2) Detection of samples on machine (Octet Data Acquisition 11.1.0.11): First, samples were added to a 96-well plate (Greiner bio-one, 655209) with 200 µL/well according to the following table. The software parameters were then set, the plate temperature was set at 30°C, and the frequency of standard kinetic signals was collected at 5.0 Hz. Next, the CH1 sensor (Fortebio, 18-0039) with 1×PBST was pre-wet for10 minutes, and then tested on the machine. Each cycle includes the following steps: 1) immersing in buffer for 60 s; 2) detecting whether the antigen non-specifically binds to the sensor; 3) regenerating with glycine solution at 10 mM pH 1.7; 4) immersing in buffer for 60s; 5) immobilizating the antibody on the sensor for 30 s; 6) immersing the sensor in the buffer for 180 s; 7) Association of the antigen- antibody for 180 s; 8) dissociation of the antigen-antibody for 10 min; 9) regenerating the sensor.
(3) Data analysis: Fortebio's Data Analysis 11.1 software was used to measure the association rate (Kₐ) and the dissociation rate (K_{d}) of the antigen-antibody in a 1:1 binding manner to calculate the equilibrium dissociation constant (K_{D}) of the antibody, and the results are shown in Table 2.

### Example 3 Detection of antibody physical and chemical properties

### 3.1 SEC- HPLC purity analysis

(1) The sample was diluted to 1 mg/mL, mixed well, centrifuged at 12,000 rpm for 5 min, followd by obtaining the supernatant and transferring it to a sample bottle, before putting it into the HPLC sample tray. The chromatographic conditions werer set as follows:

| Chromatographic conditions | parameter |
|---|---|
| Column | TSK G3000SWxl |
| Detection wavelength | 280 nm |
| Column temperature | 25 °C |
| Sample tray temperature | 5°C |
| Flow rate | 0.5 ml /min |
| Injection volume | 10µg _ |

(2) After the column was equilibrated with mobile phase (200mM phosphate buffer, pH 6.8), the sample was injected and analyzed, and the data was analyzed by chromatography software, and the peak area percentage of each peak was calculated by the peak area normalization method.

### 3.2 HIC-HPLC analysis

(1) The sample was diluted to 1 mg/ml, mixed well, centrifuged at 12,000 rpm for 5 minutes, followed by obtaining the supernatant and transferring it to the sample bottle before putting it into the HPLC sample tray. The chromatographic conditions were set as follows:

| Chromatographic conditions | parameter |
|---|---|
| Column | MAb Pac HIC- 10 |
| Detection wavelength | 214 nm |
| column temperature | 25°C |
| Sample tray temperature | 5 °C |
| flow rate | 0.8ml/min |
| Injection volume | 10µg _ |

(3) Gradient elution analysis was performed with mobile phase A (50mM phosphate buffer/1M ammonium sulfate, pH 7.0) and mobile phase B (50mM phosphate buffer, pH 7.0), and data analysis was performed by chromatography software. Based on retention time, the hydrophilic coefficient of each sample was calculated.

### 3.3 Melting temperature (Tm) value analysis

The sample to be tested was diluted with sample buffer to 1mg/mL, then 13µL of the test solution was added to the PCR tube according to the instructions of the Protein Thermal Shift^{™} Starter Kit (Cat. No. 4461146), followd by adding 5µL Protein Thermal shift TM Buffer and 2µL 10× Staining solution to make 20µLof the reaction volume, before mixing, centrifuging at 12000rpm for 5mins to remove air bubbles. The sample to be tested was placed in the PCR machine, analyzed, and the Tm value of the sample was recorded.

### 3.4 ICIEF analysis

The sample solution was added to the following well-mixed system: 1% methylcellulose (Cat. No. 101876) 70µl, urea (Cat. No. U6504) 4M, ampholyte Pharmalyte pH 3-10 (Cat. No. 17-0456- 01) 8µl, 2µl each of pI marker 5.5 (Cat. No. A58325) and 9.5 (Cat. No. A358357). An appropriate volume of ultrapure water was added to 200µl and mixed well. All samples were centrifuged at 6000 rpm for 3 min to remove air bubbles, and then the supernatant was transferred to a sample bottle, placed on a sample tray, and the sample position was recorded. After starting the operation, importing the result file into Chrom Perfect software for spectrum integration processing and calculating the isoelectric point of each peak and the percentage of each peak. The hysical and chemical test results of the antibodies are shown in Table 8.

**Table 8 Physical and chemical properties of antibodies after humanization and PTM mutation**

| Antibody name | light chain mutation | heavy chain mutation | Yield (mg/L) | Affinity KD | Thermal stability Tm | Hydrophilic HIC | Monomer rate SEC |
|---|---|---|---|---|---|---|---|
| AB1904 HzL0H0 | none | none | 132 | 3.86E-10 | 88.6 | 1.0 | |
| AB1904 L1p1H3 p2 | D30bE, E55Q, V104L | I37V,A49S,S50V, D54A,N60A,P61 D,I67F | 280 | 4.86E-10 | 82.5 | 0.94 | 99.92% |
| AB1904 L13p1H 3p3 | D30bE,E55Q, D94Y,V104L | 137V,A49S,S50V, D54G,N60A,P61 D,I67F | 282 | 3.96E-10 | 85.1 | 0.99 | 99.56% |
| AB1904 L2p1H4 p2 | D30bE, E93N, V104L | I37V,A49S,S50V, D54A,N60A,P61 D,I67F,D73N,F7 8L | 126 | 1.46E-09 | 78.1 | - | 99.73% |
| AB1904 L2p1H4 p3 | D30bE, E93N, V104L | I37V,A49S,S50V, D54G,N60A,P61 D,I67F,D73N,F7 8L | 313 | 1.67E-09 | 77.3 | - | 99.80% |
| AB1904 L1p1H5 p2 | D30bE, E55Q, V104L | I37V,A49S,S50V, D54A,N60A,167 F,F78L | 225 | 1.75E-09 | 82.2 | - | 99.78% |
| AB1904 L2p1H5 p2 | D30bE, E93N, V104L | 137V,A49S,S50V, D54A,N60A,167 F,F78L | 207 | 2.41E-09 | 81.3 | - | 99.86% |
| AB1904 L12p1H 5p2 | D30bE, E55Q, E93N, V104L | I37V,A49S,S50V, D54A,N60A,167 F,F78L | 196 | 3.37E-09 | 81.7 | - | 99.87% |
| AB1904 L13p1H 5p2 | D30bE,E55Q, D94Y,V104L | 137V,A49S,S50V, D54A,N60A,167 F,F78L | 228 | 2.08E-09 | 85.5 | - | 99.79% |
| AB1904 L1p1H5 p3 | D30bE, E55Q, V104L | 137V,A49S,S50V, D54G,N60A,I67 F,F78L | 250 | 3.61E-09 | 82.1 | - | 99.85% |
| AB1904 L2p1H5 p3 | D30bE, E93N, V104L | 137V,A49S,S50V, D54G,N60A,I67 F,F78L | 220 | 2.04E-09 | 80.7 | - | 99.87% |
| AB1904 L12p1H 5p3 | D30bE, E55Q, E93N, V104L | 137V,A49S,S50V, D54G,N60A,I67 F,F78L | 231 | 3.79E-09 | 81.3 | - | 99.89% |
| AB1904 L13p1H 5p3 | D30bE,E55Q, D94Y,V104L | 137V,A49S,S50V, D54G,N60A,I67 F,F78L | 233 | 1.86E-09 | 85.6 | - | 99.75% |

The results show that AB1904L1p1H3p2 (light chain variable region SEQ ID NO: 23, heavy chain variable region SEQ ID NO: 51) has higher affinity, and at the same time, it also removes potential post-translational modification sites, further improving chemical stability.

### Example 4 Yeast Affinity Maturation for New Antibodies

AB1904L1P1H3P2 was selected for yeast affinity maturation, and the antibodies AB1904Am1 to AB1904Am15 s shown in Table 9 were obtained, wherein the YTE (M252Y/S254T/T256E) engineered mutation was performed on the Fc fragment, which were named AB1904Am13, AB1904Am14, and AB1904Am15, respectively.

**Table 9 Affinity matured and YTE engineered mutated antibodies**

| Antibody number | light chain variable region (SEQ ID NO) | heavy chain variable region (SEQ ID NO) |
|---|---|---|
| AB1904Am1 | 24 | 52 |
| AB1904Am2 | 25 | 53 |
| AB1904Am3 | 26 | 54 |
| AB1904Am4 | 27 | 51 |
| AB1904Am5 | 28 | 51 |
| AB1904Am6 | 29 | 55 |
| AB1904Am7 | 30 | 51 |
| AB1904Am8 | 31 | 55 |
| AB1904Am9 | 32 | 51 |
| AB1904Am10 | 32 | 54 |
| AB1904Am11 | 33 | 54 |
| AB1904Am12 | 34 | 56 |
| AB1904Am13 | 26 | 54 |
| AB1904Am14 | 29 | 55 |
| AB1904Am15 | 32 | 54 |

The physical and chemical properties of the antibodies in Table 9 were tested using the method of Example 3, and the results are shown in Table 10.

**Table 10 Physical and chemical properties of antibodies after affinity maturation and YTE engineered mutations**

| Antibody number | Yield (mg/L) | Monomer purity % | Hydrophilic coefficient | pI | Acidic peak (%) | Main peak (%) | Basic peak (%) | Fab Tm (°C) |
|---|---|---|---|---|---|---|---|---|
| omalizumab | NA | 99.6 | 1 | 7.5 | 12 | 80.5 | 7.5 | 89.1 |
| AB1904Am2 | 158 | 99.8 | 0.88 | 8.3 | 56.2 | 35.9 | 8 | 84.8 |
| AB1904Am3 | 339 | 99.7 | 0.98 | 7.8 | 6.2 | 81.4 | 12.4 | 82.5 |
| AB1904Am5 | 251 | 99.8 | 0.91 | 7.5 | 69 | 21 | 10 | 84.3 |
| AB1904Am10 | 271 | 99.8 | 0.97 | 8 | 6.6 | 81 | 12.5 | 84.3 |
| AB1904Am11 | 278 | 99.7 | 0.97 | 7.8 | 6.9 | 80.8 | 12.3 | 83.2 |
| AB1904Am12 | 224 | 99.8 | 0.87 | 8 | 7.2 | 82.4 | 10.4 | 84.5 |
| AB1904Am13 | 224 | 99.7 | 0.97 | 7.4 | 7.5 | 80.9 | 11.7 | 82.4 |
| AB1904Am14 | 199 | 99.5 | 0.98 | 7.5 | 8.4 | 79.9 | 11.7 | 84 |
| AB1904Am15 | 244 | 99.6 | 0.96 | 7.6 | 7.4 | 80.8 | 11.8 | 84 |

### Example 5 The antibody binds to human IgE CH3-CH4, human full-length IgE and cynomolgus monkey IgE CH3-CH4 (Octet Red detection)

### 5.1 The antibodies described herein are capable of binding human IgE CH3-CH4

The affinity of antibody AB1904Am 1- AB1904Am 12 to human IgE CH3-CH4 (hlgE CH3-CH4, Sino Biological, Cat. No.: 29702-H08H) was determined by Octet RED96e (Fortebio). Both antigen and antibody were diluted using 1×PBST (1×PBS: Sango, B548117-0500; 0.02% Tween 20: sigma-alorich, P1379), the antigen was used at a concentration of 50 nM and the antibody was used at a concentration of 33.3 nM. On-machine detection was carried out according to the method of Example 2. The results are shown in Table 11, indicating that the antibody has high affinity to human IgE CH3-CH4, and the K_{D} value is similar to or lower than that of omalizumab.

**Table 11 Results of binding affinity of antibodies to human IgE CH3 - CH4**

| Antibody | *K_{D}* (M) | kon(1/Ms) | kdis(1/s) |
|---|---|---|---|
| AB1904Am1 | 3.117E-10 | 6.47E+05 | 2.02E-04 |
| AB1904Am2 | 3.39E-11 | 3.99E+05 | 1.35E-05 |
| AB1904Am3 | 7.695E-11 | 9.10E+05 | 7.00E-05 |
| AB1904Am4 | 1.536E-10 | 7.73E+05 | 1.19E-04 |
| AB1904Am5 | 1.328E-10 | 5.60E+05 | 7.44E-05 |
| omalizumab | 2.12E-10 | 8.64E+05 | 1.83E-04 |
| AB1904Am6 | 2.396E-10 | 8.72E+05 | 2.09E-04 |
| AB1904Am7 | 2.615E-10 | 4.98E+05 | 1.30E-04 |
| AB1904Am8 | 2.126E-10 | 5.82E+05 | 1.24E-04 |
| AB1904Am9 | 1.935E-10 | 8.19E+05 | 1.59E-04 |
| AB1904Am10 | 1.16E-10 | 8.54E+05 | 9.91E-05 |
| AB1904Am11 | 1.073E-10 | 8.51E+05 | 9.13E-05 |
| AB1904Am 12 | 1.601E-10 | 4.69E+05 | 7.51E-05 |

### 5.2 The antibodies described herein are capable of binding human full-length IgE

The affinity of the antibody to human full-length IgE (FL hIgE, ABBIOTEC, Cat. No. 250205) was determined according to the method of Example 2. The results are shown in Table 12. All of the antibodies can bind to human full-length IgE with high affinity.

**Table 12 Results of binding affinity of antibodies to human full-length IgE**

| Antibody | *K_{D}* (M) | kon(1/Ms) | kdis(1/s) |
|---|---|---|---|
| AB1904Am2 | 7.917E-10 | 4.04E+05 | 3.20E-04 |
| AB1904Am10 | 5.839E-10 | 8.25E+05 | 4.82E-04 |
| AB1904Am15 | 5.905E-10 | 8.53E+05 | 5.04E-04 |
| AB1904Am5 | 8.082E-10 | 5.70E+05 | 4.61E-04 |
| AB1904Am6 | 9.058E-10 | 9.27E+05 | 8.39E-04 |
| AB1904Am14 | 9.888E-10 | 9.42E+05 | 9.32E-04 |
| AB1904Am3 | 4.168E-10 | 9.49E+05 | 3.96E-04 |
| AB1904Am13 | 4.828E-10 | 9.52E+05 | 4.60E-04 |

### 5.3 The antibodies described herein are capable of binding cynomolgus monkey IgE CH3-CH4

Affinity of the antibodies with biotinylated cynomolgus monkey IgE CH3-CH4 (Biotin - cyno IgE CH3-CH4, Kactus Biosystems) was tested according to the method of Example 2, and the results are shown in Table 13. All of the antibodies were able to bind to biotinylated cynomolgus monkey IgE CH3-CH4 with high affinity.

**Table 13 Results of binding affinity of antibodies to biotinylated cynomolgus monkey IgE CH3-CH4**

| Antibody | *K_{D}* (M) | kon(1/Ms) | kdis(1/s) |
|---|---|---|---|
| AB1904 Am2 | 1.1E-10 | 2.06E+06 | 2.22E-04 |
| AB1904 Am3 | 1.6E-10 | 1.70E+06 | 2.72E-04 |
| AB1904 Am5 | 1.5E-10 | 1.77E+06 | 2.67E-04 |
| AB1904 Am10 | 1.7E-10 | 1.52E+06 | 2.54E-04 |
| AB1904 Am11 | 1.7E-10 | 1.50E+06 | 2.48E-04 |
| AB1904 Am6 | 1.8E-10 | 1.74E+06 | 3.08E-04 |
| AB1904Am15 | 1.005E-10 | 2. 10E+06 | 2.11E-04 |

### Example 6 The antibody binds to human full-length IgE and cynomolgus monkey IgE CH3-CH4 (Biacore detection)

First, the Biotin CAPture reagent was captured on the CAP chip (GE Healthcare, Cat. No. 28-9202-34) at a flow rate of 2 µL/min for 300s. HBS-EP+ was used as experimental buffer. Binding of a certain concentration of antibody to the captured antigen was determined for each injection cycle, wherein each cycle include capture of antigen, injection of different concentrations of antibody, and regeneration. 2 µg/ml of biotinylated human full-length IgE (Biotin-FL hIgE, Abbiotec, Cat. No. 250205, biotinylated by ourselves) or 0.25 µg/ml of biotinylated Cynomolgus monkey IgE CH3-CH4 (Biotin-cyno IgE, customized by Kactus Biosystems) were captured on 2 channel at a flow rate of 10 µL/min for 60 s, and 1 channel was used as a blank reference channel. At a flow rate of 30 µL/min, serially diluted antibodies (100 nM, 50 nM, 25 nM, 12.5 nM, 6.25 nM, 3.125 nM, 1.5625 nM, 0 nM) were sequentially injected into channels 1 and 2, and the association and disassociation were determined. Chip regeneration was performed with regeneration buffer (6 M guanidine hydrochloride, 0.25 M sodium hydroxide) at a flow rate of 10 µL/min to remove captured antigens and antibodies. Data were analyzed using Biacore 8K analysis software. The model used for software analysis is for 1:1 binding.

The results shown in Table 14 indicate that all the antibodies described in this application were capable of binding to both biotinylated human full-length IgE and cynomolgus monkey IgE CH3 - CH4, with a K_{D} value non-significantly different from or less than that of omalizumab..

**Table 14 Results of binding affinity of antibodies to biotinylated human full-length IgE and cynomolgus monkey IgE CH3 - CH4**

| Antigen | Antibody | ka (1/Ms) | kd (1/s) | K_{D} (M) |
|---|---|---|---|---|
| Biotin-human full-length IgE | AB1904Am10 | 2.10E+05 | 7.79E-04 | 3.71E-09 |
| | AB1904Am15 | 2.54E+05 | 7.99E-04 | 3.15E-09 |
| | omalizumab | 3.48E+05 | 2.16E-03 | 6.20E-09 |
| Biotin-Monkey IgE CH3-CH4 | AB1904Am10 | 4.29E+05 | 1.42E-03 | 3.32E-09 |
| | AB1904Am15 | 4.27E+05 | 1.46E-03 | 3.41E-09 |
| | omalizumab | 6.48E+05 | 2.49E-03 | 3.85E-09 |

### Example 7 The antibodies binds to human and cynomolgus FcRn (detected by Octet Red)

The affinity of the antibody to human FcRn (Aero, FCN-H52W7) and cynomolgus monkey FcRn (Aero, FCM-C52W9) was determined according to the method of Example 2 using Octet RED96e (Fortebio). Wherein, human FcRn and cynomolgus FcRn were diluted to 5 µg/mL with 1×PBS (10 mMNa2HPO4.12H2O, 2 mM KH2PO4, 137 mMNaCl, 2.7 mM KCl, pH 6.0), respectively, and biotin-conjugated THETM His-tagged mouse monoclonal antibody was diluted to 2 µg/mL; the antibody to be tested was diluted with 1× PBST (1×PBS, 0.02% Tween 20, pH6.0), and the antibody dilution concentration ranged from 1000 nM - 31.3 nM. The results are shown in Table 15. All the antibodies were capable of binding to cynomolgus monkey and human FcRn.

**Table 15 Results of affinity of antibodies with cynomolgus monkey and human FcRn**

| Antibody | KD (M) | kon(1/Ms) | kdis(1/s) | FcRn |
|---|---|---|---|---|
| AB1904Am10 | 9.139E-08 | 1.16E+06 | 1.06E-01 | Cynomolgus FcRn |
| AB1904Am15 | 3.154E-08 | 9.22E+05 | 2.91E-02 | |
| AB1904Am10 | 9.866E-08 | 1.22E+06 | 1.21E-01 | hFcRn |
| AB1904Am15 | 3.283E-08 | 8.75E+05 | 2.87E-02 | |

### Example 8 Antibody blocks binding of biotinylated human full-length IgE to FcεRI

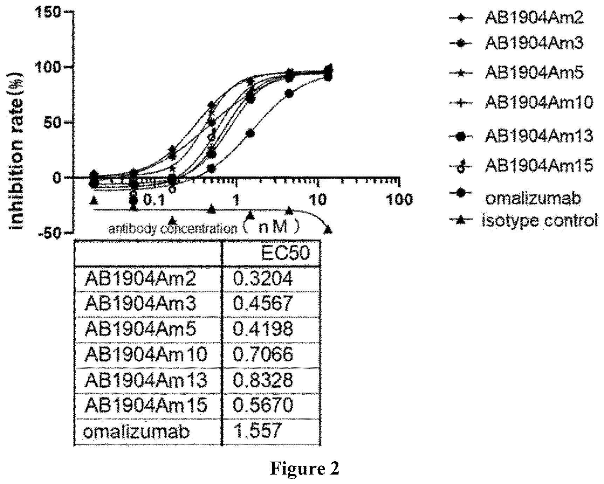

FcεRIα (R&D, 6678-Fc) was diluted to 0.5 µg/ml in coated enzyme labeling plate with 100 µl/well using PBS, the sealing film was sticked before incubation at 37°C for 1 hour, followed by washing the plate 3 times with washing solution (PBS + 0.05 % TWEEN-20). Then, a blocking solution (PBS + 0.05% TWEEN-20 + 2% BSA) was prepared with washing solution, 300 µl of blocking solution was added to each well, and incubation at 37°C for 1 hour. Biotinylated human full-length IgE (Biotin-FL hIgE, Abbiotec, Cat. No. 250205, biotinylated in-house) with a concentration of 160 ng/ml was prepared in blocking solution; then anti-IgE antibody was prepared in blocking solution, initial concentration starting at 4 µg/ml, 3-fold serial dilution (dilution 7 concentration points + 1 0 concentration point); then 60 µL of anti-IgE antibody was added to 60 µL Biotin-FL hIgE, and incubated at 37°C 2 hours. Then the blocked ELISA plate was taken out, washed by washing solution three times, and anti-IgE antibody and biotinylated human full-length protein complex solution that was pre-incubated at 37°C was added to 100µl/well, and then incubated at 37°C for 1 hour. The plate was washed with washing solution 3 times, and after SA-HRP (sigma, S2438-250UG) was diluted with blocking solution in 1/5000, it is added to the ELISA plate at 100 µl/well, incubation at 37 °C for 1 hour; the plate was washed 3 times with washing solution, and then TMB chromogenic solution (Biopanda, product number: TMB-S-003) was added at 100 µl/well, reaction at room temperature in the dark for 10 minutes, followed by adding stop solution (Solarbio, product number: C1058) at 50 µl/well to stop the reaction before determination of the absorbance value at a wavelength of 450nm. The results as shown in Figure 2 indicate that the antibodies described in the present application were capable of blocking the binding of biotinylated IgE to FcεRI, and the EC₅₀ value is lower than that of omalizumab.

### Example 9 Antibody blocks binding of biotinylated human full-length IgE to CD23 on IM-9 cell surface

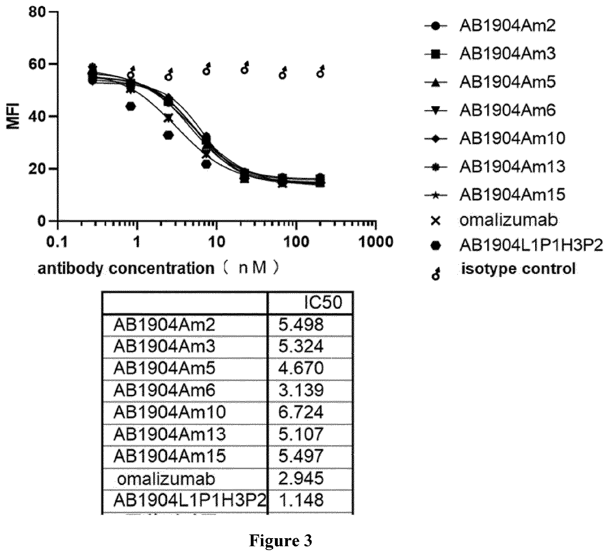

IM-9 cells were cultured using RPMI1640 + 10% FBS (RPMI1640, FBS, Thermo Fisher) at 37°C, 5% carbon dioxide. IM-9 cells were collected, washed once with FACS buffer (PBS+2% FBS), and resuspended in FACS buffer solution at a cell density of 2.5^{∗}10⁶ cells/ml. Cell suspension (100 µl per well) was added into U-bottom 96-well cell culture plate, and then placed in a 4-degree refrigerator for 30 min. The biotinylated human full-length IgE (hlgE, ABBIOTEC, Cat. No. 250205, biotinylated in-house) was prepared in FACS buffer soution at a concentration of 20 µg/ml; then anti-IgE antibodies were prepared using FACS buffer at a starting concentration of 60 µg/ml, 3-fold serial dilution (diluted to seven concentration points + one 0 concentration point), then 60 µL of the anti-IgE antibody was added to 60 µL of biotin- FL hIgE, followed by incubation at 4 °C for 1 hour. At the same time, 2.5 µL of Fc Block (BD Pharmingen, Cat. No. 564220) was added to each well of cells, and incubated at 4 °C for 1 hour; the 96-well cell culture plate was taken out of the refrigerator at 4 °C, centrifuged at 2000 rpm for 5 minutes, before the supernatant was discarded, and then the anti-IgE antibody and the biotinylated human full-length IgE protein complex solution that was pre-incubated was added at 100 µl/well, incubated at 4°C for 1 hour, centrifuged at 2000 rpm for 5 min, followed by discarding the supernatant, and washing three times with FACS buffer solution. After FITC -coupled streptavidin (Invitrogen, Cat. No: SA1001) was diluted in 1:500, added 100 µL to each well, incubated at 4 degrees for 1 hour, centrifuged at 2000 rpm for 5 min, followed by discarding the supernatant, before washed with FACS buffer solution for 3 times; The cells were then diluted with FACS buffer solution in 1:2, and detected by using a Guava easyCyte 6-2L Benchtop Flow Cytometer. The results as shown in Figure 3 indicate that the antibodies described in the present application were capable of blocking the binding of biotinylated human full-length IgE to CD23 on the surface of IM-9 cells.

### Example 10 Antibody blocks biotinylated human full-length IgE-stimulated histamine release from FcεRI/RBL-2H3

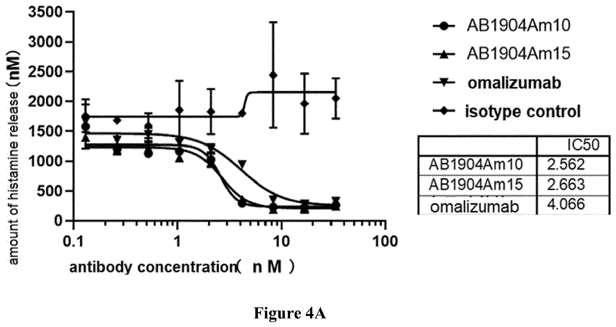

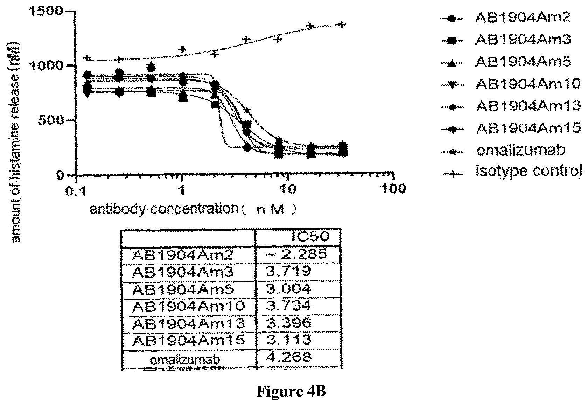

FcεRI/RBL-2H3 cells (constructed by the Genechem gene) were cultured under the conditions of 37°C, 5% carbon dioxide using FcεRI/RBL-2H3 medium (MEM + 15% FBS + 1 mM sodium pyruvate + 1 µg/ml Puromycin). Human full-length IgE protein (ABBIOTEC, Cat. No: 250205) with a concentration of 2.4 µg/ml was prepared using cell culture medium; then the anti-IgE antibody was prepared by using cell culture medium, starting at a concentration of 15 µg/ml, 2 -fold serial dilution (nine concentration points + one 0 concentration). 50 µL of human full-length IgE protein was first added to a 96 -well flat-bottomed cell culture plate, and then 50 µL of the prepared anti-IgE antibody. At the same time, FcεRI/RBL-2H3 cells were collected, resuspended in medium at the cell concentration of 2^{∗}10⁶ cells/ml, and added to the corresponding 96 -well flat-bottom cell culture plate in 50 µl/ well, and then the cell culture plate was incubated in cell incubator overnight at 37°C, 5 % carbon dioxide. The next day, after the supernatant was aspirated and discarded, the cells were washed twice using Assay Buffer (Tyrode's buffer (130 mM NaCl, 5 mM KCl, 10 nM HEPES, 1.4 mM CaCl2, 1 mM MgCl2.6H2O, 5.6 mM Glucose, 0.1%BSA), pH7.4), followed by addition of 200 µl of 1 µg/ml polyclonal anti-IgE antibody (R&D, Cat. No.: G-107-C) to each well, before incubation in a cell incubator at 37°C, 5% carbon dioxide for 30 minutes; the supernatant was drawn and detected using Histamine Dynamic kit (CISBIO, product number: 62HTMDPEG) according to the detection method based on the kit instructions. The results are shown in Figure 4A and Figure 4B, showing that the antibodies were capable of inhibiting the FL hIgE induced histamine release from FcεRI/RBL-2H3 induced, and the IC ₅₀ value is lower than that of omalizumab, indicating that the inhibition effect is superior than that of omalizumab.

### Example 11 Human whole blood histamine release assay

Volunteers should not take allergy-causing drugs, antihistamines, oral corticosteroids, and any substance that blocks H2 receptors 24 hours before donating blood. Whole blood was treated with heparin as an anticoagulant.

Histamine release was performed according to the operation steps of the kit (Sigma, Cat. No.: IB89145), as follows: the antibody to be tested was diluted with PBS to 6000, 600, 60 and 6 nM, the positive control anti-IgE serum in the kit was diluted by 1000 folds with histamine release buffer solution before use. Loading samples and operations to perform the histamine release process were shown in Table 16 below.

**Table 16 Instructions for loading samples of histamine release**

| | Antibody samples | positive control | spontaneo us release | total histamine release |
|---|---|---|---|---|
| Histamine release buffer | - | - | 100µL | 180µL |
| Antibody | 100µL | - | - | - |
| anti-IgE serum | - | 100µL | - | - |
| Heparin anticoagulated human whole blood | 100µL | 100µL | 100µL | 20µL |
| After mixing, incubate at 37 degrees for 60 min | | | | After mixing, incubate at 90 degrees for 10 min |
| After 10 min in ice bath, centrifuge at 700 g for 10 min (brake off) and take 50 µl of supernatant for acetylation. | | | | |

The histamine ELISA was performed according to the operation steps of the kit (Sigma, Cat. No: IB89128), as follows. 50 µl of the supernatant or the standard and control substances was diluted by 1-fold with deionized water, and added to the corresponding wells respectively. Then, 25 µl of acetylation buffer solution and 25 µl of acetylation solution were added to each well and shaken at 600 rpm for 45 minutes at room temperature. Finally, 100 µl of deionized water was added, and shaken at 600 rpm for 15 minutes at room temperature. After the sample was acetylated, 25 µl was added to the histamine-coated plate, and then 100 µl of antihistamine serum was added, followed by sealing with a sealing film before shaking at 600 rpm for 3 hours at room temperature. After incubation, the liquid in the well plate was discarded, followed by adding 300 µl of washing solution to each well for washing 4 times, before adding 100 µl of enzyme-linked secondary antibody to each well, and then shaking at 600 rpm for 30 minutes at room temperature. The liquid in the well plate was discarded, followed by adding 300 µl washing solution to each well for washing 4 times, before adding 100 µl substrate to each well, and then shaking at 600 rpm for 30 minutes at room temperature. Finally, 100 µl of stop solution was added to stop the reaction, and the absorbance values at 450 nm and 570 nm were collected within 10 minutes using an Envision microplate reader (PerkinElmer).

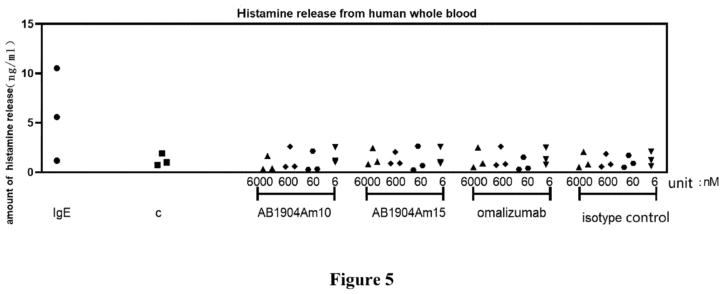

The results are shown in Figure 5, showing that Anti -IgE serum was capable of cross-linking or activating IgE that had bound to FcεRI, and inducing histamine release, while omalizumab, AB1904Am10 and AB1904Am15 do not.

### Example 12 Antibody Stability Studies

The stability of candidate antibodies is comprehensively evaluated through stability experimental conditions of high temperature, low pH, and repeated freeze-thaw. The key test items for the stability study are mainly aimed at the key purity indicators of the product, including SEC-HPLC, iCIEF, and non-reducing CE-SDS.

### 11.1 High temperature stability test

This study focused on investigating the stability of candidate antibodies under high temperature conditions. The samples were placed under the experimental conditions of 40 + 2°C, and detected for SEC-HPLC purity, charge isomer purity, nrCE-SDS purity on the 0th, 3rd, 7th, 14th, 21st and 28th days. The experimental results are as follows:

**Table 17 Results of antibody stability at high temperature**

| High temperature 40°C | | omalizumab | | | | | | AB1904Am10 | | | | | | AB1904Am15 | | | | | |
|---|---|---|---|---|---|---|---|---|---|---|---|---|---|---|---|---|---|---|---|
| Test items | | 0 days | 3 days | 7 days | 14 days | 21 days | 28 days | 0 | 3 days | 7 days | 14 days | 21 days | 28 days | 0 days | 3 days | 7 days | 14 days | 21 days | 28 days |
| SEC-HPLC | Aggregate % | 0.78 | 0.88 | 0.93 | 0.95 | 1.01 | 1.08 | 0.72 | 0.56 | 0.55 | 0.63 | 0.66 | 0.80 | 0.64 | 0.47 | 0.48 | 0.47 | 0.53 | 0.67 |
| | Monomer % | 98.86 | 98.21 | 97.64 | 96.70 | 95.80 | 94.88 | 98.78 | 98.39 | 97.87 | 96.78 | 95.79 | 94.71 | 99.36 | 98.47 | 97.88 | 96.99 | 96.01 | 94.99 |
| | Fragment % | 0.36 | 0.91 | 1.43 | 2.35 | 3.20 | 4.04 | 0.5 | 1.05 | 1.58 | 2.59 | 3.55 | 4.49 | 0 | 1.05 | 1.64 | 2.54 | 3.45 | 4.34 |
| NRCE-SDS | Main peak % | 97.7 | 94.9 | 93.8 | 92.1 | 90.4 | 87.3 | 97.0 | 93.1 | 91.7 | 91.1 | 90.0 | 86.1 | 96.6 | 94.3 | 93.7 | 92.4 | 91.7 | 89.1 |
| | Fragment % | 2.3 | 5.1 | 6.2 | 7.9 | 9.7 | 12.5 | 3.1 | 6.9 | 8.2 | 9.0 | 100 | 13.9 | 3.5 | 5.6 | 6.4 | 7.6 | 8.3 | 10.9 |
| iC IEF | Acid peak % | 9.0 | 10.8 | 13.4 | 20.5 | 26.0 | 31.7 | 7.6 | 12.3 | 15.8 | 21.2 | 26.3 | 31.5 | 8.8 | 11.3 | 17.9 | 24.8 | 30.9 | 35.6 |
| | Main peak % | 80.6 | 69.6 | 62.8 | 55.0 | 49.8 | 45.6 | 84.3 | 77.4 | 72.8 | 66.8 | 61.5 | 55.0 | 82.6 | 78.2 | 70.6 | 63.1 | 57.6 | 52.8 |
| | Basic peak % | 10.4 | 19.6 | 23.8 | 24.6 | 24.2 | 22.7 | 8.1 | 10.3 | 11.4 | 12.0 | 12.2 | 13.5 | 8.7 | 10.5 | 11.4 | 12.1 | 11.6 | 11.7 |

### 11.2 Low pH Stability Test

The stability of candidate antibodies under low pH conditions was mainly investigated, wherein the pH of the sample was adjusted to 5.0 with hydrochloric acid, placed at 40 + 2°C, and detected for SEC-HPLC purity, charge isomer purity, nrCE-SDS purity at 0h, 6h, 24h, 48h, 72h, respectively. The experimental results are as follows:

**Table 18 Results of antibody stability at low pH**

| Low pH 5.0, 40°C | | omalizumab | | | | | AB1904Am10 | | | | | AB1904Am15 | | | | |
|---|---|---|---|---|---|---|---|---|---|---|---|---|---|---|---|---|
| Test items | | 0 hour | 6 hours | 24 hours | 48 hours | 72 hours | 0 hour | 6 hours | 24 hours | 48 hours | 72 hours | 0 hour | 6 hours | 24 hours | 48 hours | 72 hours |
| SEC-HPLC | Monomer % | 98.86 | 98.39 | 98.56 | 98.26 | 98.12 | 98.78 | 98.71 | 98.55 | 98.59 | 98.53 | 99.36 | 98.96 | 98.83 | 98.72 | 98.71 |
| | Aggregate % | 0.78 | 1.11 | 0.91 | 1.00 | 0.99 | 0.72 | 0.67 | 0.73 | 0.63 | 0.56 | 0.64 | 0.53 | 0.53 | 0.58 | 0.48 |
| | Fragment % | 0.36 | 0.50 | 0.52 | 0.73 | 0.89 | 0.5 | 0.62 | 0.72 | 0.78 | 0.91 | 0 | 0.51 | 1.10 | 0.71 | 0.80 |
| NRCE-SDS | Main peak % | 97.7 | 97.5 | 97.0 | 96.8 | 96.6 | 97.0 | 97.2 | 96.7 | 96.6 | 96.5 | 96.6 | 97.1 | 96.8 | 96.1 | 96.0 |
| | Fragment % | 2.3 | 2.5 | 3 | 3.2 | 3.4 | 3.1 | 2.9 | 3.3 | 3.3 | 3.5 | 3.5 | 3 | 3.3 | 3.9 | 4 |
| iCIEF | Acid peak % | 9.0 | 9.7 | 8.7 | 8.7 | 9.2 | 7.6 | 8.1 | 8.7 | 9.8 | 10.6 | 8.8 | 9.0 | 9.7 | 10.7 | 11.9 |
| | Main peak % | 80.6 | 78.2 | 72.8 | 66.4 | 60.6 | 84.3 | 82.6 | 81.1 | 79.1 | 78.0 | 82.6 | 81.6 | 80.1 | 78.6 | 76.7 |
| | Basic peak % | 10.4 | 12.1 | 18.5 | 24.8 | 30.2 | 8.1 | 9.3 | 10.2 | 11.1 | 11.4 | 8.7 | 9.4 | 10.2 | 10.7 | 11.4 |

### 11.3 Repeated freeze-thaw stability test

The stability of the candidate antibody under repeated freezing and thawing conditions was mainly investigated, wherein a cycle was from when the sample is frozen at -70±10°C to when it is thawed at room temperature. Samples were tested in the third cycle and the sixth cycle, respectively. The experimental results are as follows:

**Table 19 Results of antibody stability under repeated freeze-thaw cycles**

| Repeated freezing and thawing | | omalizumab | | | AB1904Am10 | | | AB1904Am15 | | |
|---|---|---|---|---|---|---|---|---|---|---|
| Test items | | 0 times | 3 times | 6 times | 0 times | 3 times | 6 times | 0 times | 3 times | 6 times |
| SEC-HPLC | Monomer % | 98.86 | 98.71 | 98.54 | 98.78 | 98.80 | 98.83 | 99.36 | 98.92 | 98.76 |
| | Aggregate % | 0.78 | 0.86 | 1.01 | 0.72 | 0.64 | 0.60 | 0.64 | 0.48 | 0.54 |
| | Fragment % | 0.36 | 0.44 | 0.46 | 0.5 | 0.57 | 0.57 | 0 | 0.60 | 0.70 |
| NRCE-SDS | Main peak % | 97.7 | 96.4 | 96.4 | 97.0 | 96.0 | 96.0 | 96.6 | 95.5 | 95.2 |
| | Fragment % | 2.3 | 3.1 | 3.3 | 3.1 | 4.0 | 4.0 | 3.5 | 4.6 | 4.8 |
| iC IEF | Acid peak % | 9.0 | 9.9 | 10.1 | 7.6 | 8.4 | 8.0 | 8.8 | 8.7 | 8.3 |
| | Main peak % | 80.6 | 79.2 | 79.2 | 84.3 | 82.4 | 83.0 | 82.6 | 82.4 | 82.6 |
| | Basic peak % | 10.4 | 10.9 | 10.8 | 8.1 | 9.2 | 9.1 | 8.7 | 9.0 | 9.1 |

The high temperature stability, low pH stability and repeated freeze-thaw stability of AB 1904Am10, AB1904Am15 and omalizumab were studied. The results of the high temperature and low pH stability studies showed that the stabilities of candidate antibodies AB1904Am10 and AB1904Am15 were superior than that of omalizumab under high temperature and low pH conditions, especially the reduction of the amount of aggregates in the SEC-HPLC detection, and the decreasement of the main peak in the iCIEF detection, indicating that the candidate antibodies AB1904Am10 and AB1904Am15 were more tolerant and more stable to temperature and low pH, thereby being more stable during production, storage and use. The results of repeated freeze-thaw stability study showed that all key quality indicators of AB 1904Am10 and AB 1904Am15 for purity had well stability after repeated freezing and thawing for 6 times.

### Example 13 Pharmacokinetic (PK) studies in a humanized FcRn mouse model

Humanized FcRn mice were used as test animals to study the pharmacokinetic indicators of the three tested drugs omalizumab, AB1904Am10 and AB1904Am15 after single subcutaneous administration. All animal experimental protocols were reviewed and approved by the IACUC. hFcRn mice were purchased from Beijing Biositu, male, 6-8 weeks old, weighing 23-26g, housed in SPF animal room, fed with standard pellet feed, eating and drinking freely, room temperature 18-24 °C, relative humidity 40% ~50%, alternating day and night for 12 hours a day. A total of 12 experimental animals were randomly divided into three groups, with four animals in each group, subcutaneously administered a single dose of 10 mg/kg, and the administration volume was 10 mL/kg. Blood collection time points are before administration, 2h, 6h, 24h (day 1), day 2, day 3, day 4, day 7, day 10, day 14, day 21, day 28, day 35 and day 42 after administration. 60 µL of whole blood was collected into EP tubes by cheek puncture, left to stand at room temperature for 30 min, and then centrifuged (2000 g, 4 °C, 5 min) to separate serum. Each sample is divided into 2 parts (test tube and backup tube), 10 µL/tube, stored at -80°C.

The pharmacokinetics of the three drugs at different time points were analyzed by Elisa indirect method. The coating antigen was anti-human IgG1 antibody (Fc specific) (Abeam, catalog number: ab1927), 2µg/ml, 100ul/well, 37°C, 2h. After washing the plate, 200ul/well of block solution was used to incubate at 4°C overnight. Serum samples were added, 50 µl/well, 37°C, 1 h. The detection antibody was mouse anti-human Fab HRP (1:10000) (Abeam, Cat. No.: ab87422), 100 µl/well, 37°C, 0.5h. TMB chromogenic solution (KPL, Cat. No: 52-00-03) was used to developed color, and the microplate reader (Molecular Devices, SpectraMax M3) was used to collect values at OD450. The drug concentration was obtained according to the standard curve, and the PK parameters were obtained by processing the date using the PK Solver non-compartmental model.

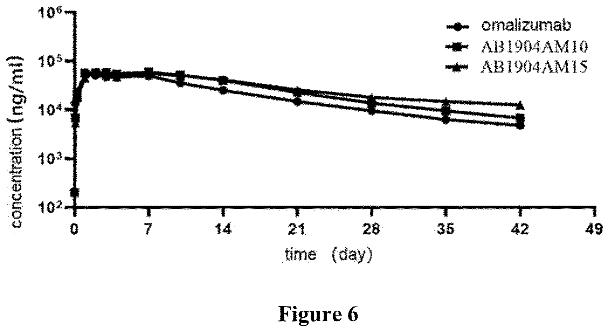

The results are shown in Figure 6. After administration of omalizumab, half-life (t1/2)=10.9 days, and the highest concentration (Cmax) was 53214.1ng/ml; AUC was 900661 ng/ml*d; after administration of AB1904AM10, half-life (t1/2)= 13.5 days, the highest concentration (Cmax) was 60441.4ng/ml; AUC was 1220787 ng/ml*d; after administration of AB1904AM15, half-life (t1/2)=26.6 days, the highest concentration (Cmax) was 57355.7ng/ml. AUC 1284785 ng/ml*d.

### Example 14 Pharmacokinetic-Pharmacodynamic (PK/PD) Study in a Cynomolgus Monkey Model

Cynomolgus monkeys were used as subject animals to study the pharmacokinetics and pharmacodynamics of the three test drugs omalizumab, AB1904Am10 and AB1904Am15 after single subcutaneous administration.

All animal experimental protocols were reviewed and approved by the IACUC. 9 SPF grade male Cynomolgus monkeys were over 4 years old, weighing 4.1-5.7kg used in the experiment, housed in SPF grade animal room, eating and drinking freely. Dosage of administration was 10 mg/kg; route of administration was subcutaneous injection; volume of administration was calculated according to the specific concentration of the drug. The cynomolgus monkeys were grouped into 3 TAs with 3 cynomolgus monkeys per TA; for administration: the administration volume was calculated according to body weight, the drug was injected subcutaneously in a single time, and record the administration time; for collection points: before administration, 2 hours, 6h, 24h (day 1), day 2, day 3, day 4, day 7, day 10, day 14, day 21, day 28, day 35, day 42 and day 56 after administration, 300 µL of whole blood per each was collected into EP tubes from the cephalic vein and saphenous vein, and the blood collection times were recorded; for processing samples: the whole blood was left to stand at room temperature for 1 h, centrifuged: 6,000 g, 25 °C, 10 min, and the serum was separated, followed by dividing each sample into 5 parts (1 test tube and 4 backup tubes), 30 µL/tube, and then stored at -80°C.

The pharmacokinetics of the three drugs at different time points were analyzed by Elisa indirect method. The coating antigen was anti-human IgG1 antibody (Fc specific) (Abeam, Cat. No.: ab1927), 2µg/ml, 100ul/well, 37°C, 2h. After washing the plate, 200ul/well blocking solution was used to incubate at 4°C overnight. Serum samples were added, 50ul/well, 37°C, 1h. The detection reagent is human IgE-Biotin (Abbiotec, Cat. No.: 250205, biotinylated labeling by ourselves) + SA-HRP (sigma, Cat. No.: S2438-250UG) and added 100ul/well, at 37°C, for 0.5h. TMB chromogenic solution (KPL, Cat. No.: 52-00-03) developed color, and the microplate reader (Molecular Devices, SpectraMax M3) was used to collect values at OD450. The drug concentration was obtained according to the standard curve, and the PK parameters were obtained by processing the date using the PK Solver non-compartmental model.

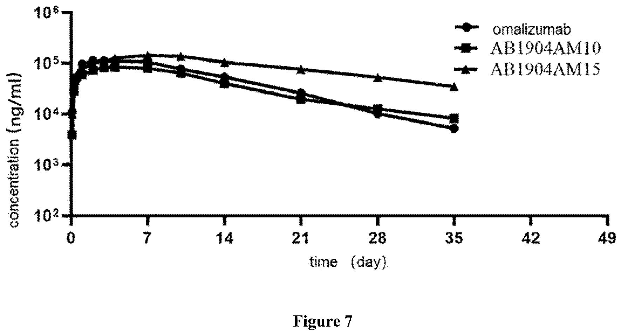

The results are shown in Figure 7. After administration of omalizumab, the half-life (t1/2)=6.35 days, the highest concentration (Cmax) was 112493 ng/ml, and the AUC was 1704601 ng/ml*d; after administration of AB1904AM10, the half-life (t1/2)=11.15 days, the highest concentration (Cmax) is 83940 ng/ml, the AUC is 1324493 ng/ml*d; after administration of AB1904AM15, the half-life (t1/2)=12.2 days, the highest concentration (Cmax) was 142880 ng/ml, and the AUC was 3061056 ng/ml*d.

Then the free IgE in serum was detected by Elisa indirect method. The coating antigen was AB1904A m15, 0.5 µg/ml, 100 ul/well, overnight at 4°C. After washing the plate, 200ul/well blocking solution was used to incubate at 37°C for 1h. Serum samples were added, with 50ul/well, at 37°C, for 1.5h. The biotin-labeled omalizumab with 500ng/ml was added to 100uL per well, for 1h; then SA-HRP (1:10000, sigma, Cat. No.: S2438-250UG) was added to 100ul/well, at 37°C, for 1h. TMB chromogenic solution (KPL, Cat. No.: 52-00-03) was used to developed color, and the microplate reader (Molecular Devices, SpectraMax M3) was used to read OD450. Free IgE concentration was obtained according to the standard curve.

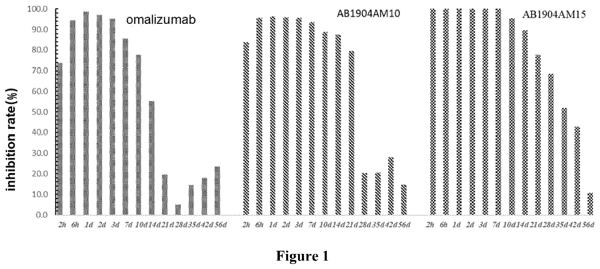

The results are shown in Figure 1. After administration of omalizumab, the recovery time for free IgE to the initial concentration was about 21 days; after administration of AB 1904Am 10, the recovery time for free IgE to the initial concentration was about 28 days; after administration of AB1904Am15, the recovery time for free IgE to the initial concentration was about 42 - 56 days.

## Claims

1. An antigen-binding protein comprising an antibody light chain variable region (VL), wherein the VL comprises amino acid mutations at one or more positions selected from the group consisting of E55 and V104 compared with an amino acid sequence as shown in SEQ ID NO: 22.

2. The antigen-binding protein of claim 1, wherein the VL comprises amino acid mutations at one or more positions selected from the group consisting of D30b, V29, D30, Y30a and G30c.

3. The antigen-binding protein of any one of claims 1-2, wherein the VL comprises amino acid mutations at positions of the following group: D30b, E55 and V104.

4. The antigen-binding protein of any one of claims 1-3, wherein the VL comprises amino acid mutations at the positions selected from any group consisting of:
(1) D30b, E55, V104, V29, D30 and Y30a;
(2) D30b, E55, V104, D30, Y30a and G30c;
(3) D30b, E55, V104 and Y30a;
(4) D30b, E55, V104, Y30a and G30c;
(5) D30b, E55, V104, D30, Y30a and G30c; and
(6) D30b, E55, V104, D30 and Y30a.

5. The antigen-binding protein of any one of claims 1-4, wherein the amino acid mutation at position E55 comprises E55Q.

6. The antigen-binding protein of any one of claims 1-5, wherein the amino acid mutation at position V104 comprises V104L.

7. The antigen-binding protein of any one of claims 2-6, wherein the amino acid mutation at position D30b comprises D30bE.

8. The antigen-binding protein of any one of claims 2-7, wherein the amino acid mutation at position V29 comprises V29 L.

9. The antigen-binding protein of any one of claims 2-8, wherein the amino acid mutation at position Y30a comprises any one selected from the group consisting of Y30A, Y30aS and Y30aD.

10. The antigen-binding protein of any one of claims 2-9, wherein the amino acid mutation at position D30 comprises any one selected from the group consisting of D30Y, D30A, D30E, D30F, D30G and D30N.

11. The antigen-binding protein of any one of claims 2-10, wherein the amino acid mutation at position G30c comprises any one selected from the group consisting of G30cA, G30cY and G30cW.

12. The antigen-binding protein of any one of claims 2-11, wherein the VL comprises amino acid mutations of the following group: D30bE, ESSQ and V104L.

13. The antigen-binding protein of any one of claims 1-12, wherein the VL comprises an amino acid sequence as shown in SEQ ID NO: 57.

14. The antigen-binding protein of any one of claims 1-13, wherein the VL comprises any one of amino acid sequences as shown in SEQ ID NOs: 23-34.

15. The antigen-binding protein of claims 1-14, comprising an antibody heavy chain variable region (VH), wherein the VH comprises amino acid mutations at one or more positions selected from the group consisting of I37, A49, S50 and D54 compared with the amino acid sequence as shown in SEQ ID NO: 50.

16. The antigen-binding protein of claim 15, wherein the VH comprises amino acid mutations at one or more positions selected from the group consisting of N60, P61, I67, T30, S31, S96 and H97.

17. The antigen-binding protein of any one of claims 15-16, wherein the VH comprises amino acid mutations at positions of the following group: N60, P61, I67, I37, A49, S50 and D54.

18. The antigen-binding protein of any one of claims 15-17, wherein the VH comprises amino acid mutations at positions selected from any group consisting of:
(1) N60, P61, I67, I37, A49, S50, D54, S96 and H97;
(2) N60, P61, I67, I37, A49, S50, D54 and T30;
(3) N60, P61, I67, I37, A49, S50, D54 and S96;
(4) N60, P61, I67, I37, A49, S50, D54 and S31; and
(5) N60, P61, I67, I37, A49, S50, D54 and H97.

19. The antigen-binding protein of any one of claims 15-18, wherein the amino acid mutation at position I37 comprises 137V.

20. The antigen-binding protein of any one of claims 15-19, wherein the amino acid mutation at position A49 comprises A49S.

21. The antigen-binding protein of any one of claims 15-20, wherein the amino acid mutation at the position S50 comprises S50V.

22. The antigen-binding protein of any one of claims 15-21, wherein the amino acid mutation at position D54 comprises D54A.

23. The antigen-binding protein of any one of claims 16-22, wherein the amino acid mutation at the position N60 comprises N60A.

24. The antigen-binding protein of any one of claims 16-23, wherein the amino acid mutation at position P61 comprises P61D.

25. The antigen-binding protein of any one of claims 16-24, wherein the amino acid mutation at position I67 comprises I67F.

26. The antigen-binding protein of claims 16-25, wherein the amino acid mutation at the position T30 comprises T30R.

27. The antigen-binding protein of any one of claims 16-26, wherein the amino acid mutation at position S31 comprises S31Q.

28. The antigen-binding protein of any one of claims 16-27, wherein the amino acid mutation at position S96 comprises S96T.

29. The antigen-binding protein of any one of claims 16-28, wherein the amino acid mutation at position H97 comprises H97N.

30. The antigen-binding protein of any one of claims 15-29, wherein the VH comprises amino acid mutations of the following group: N60A, P61D, I67F, I37V, A49S, S50V and D54A.

31. The antigen-binding protein of any one of claims 15-20, wherein the VH comprises amino acid sequence as shown in SEQ ID NO: 58.

32. The antigen-binding protein of any one of claims 15-31, wherein the VH comprises any one of amino acid sequence as shown in SEQ ID NOs: 51-56.

33. The antigen-binding protein of any one of claims 15-32, wherein the VL and the VH comprise amino acid mutations at positions selected from any group consisting of:
(1) the VL including amino acid mutations at the following positions: D30b, E55, V104, V29, D30 and Y30a; the VH including amino acid mutations at the following positions: N60, P61, I67, I37, A49, S50, D54, S96 and H97;
(2) the VL including amino acid mutations at the following positions: D30b, E55, V104, D30, Y30a and G30c; the VH including amino acid mutations at the following positions: N60, P61, I67, I37, A49, S50, D54 and T30;
(3) the VL including amino acid mutations at the following positions: D30b, E55, V104 and Y30a; the VH including amino acid mutations at the following positions: N60, P61, I67, I37, A49, S50, D54 and S96;
(4) the VL including amino acid mutations at the following positions: D30b, E55, V104 and Y30a; the VH including amino acid mutations at the following positions: N60, P61, I67, I37, A49, S50 and D54;
(5) the VL including amino acid mutations at the following positions: D30b, E55, V104, Y30a and G30c; the VH including amino acid mutations at the following positions: N60, P61, I67, I37, A49, S50 and D54;
(6) the VL including amino acid mutations at the following positions: D30b, E55, V104 and Y30a; the VH including amino acid mutations at the following positions: N60, P61, I67, I37, A49, S50, D54 and H97;
(7) the VL including amino acid mutations at the following positions: D30b, E55, V104, D30, Y30a and G30c; the VH including amino acid mutations at the following positions: N60, P61, I67, I37, A49, S50 and D54;
(8) the VL including amino acid mutations at the following positions: D30b, E55, V104, D30 and Y30a; the VH including amino acid mutations at the following positions: N60, P61, I67, I37, A49, S50, D54 and H97;
(9) the VL including amino acid mutations at the following positions: D30b, E55, V104, D30, Y30a, and G30c; the VH including amino acid mutations at the following positions: N60, P61, I67, I37, A49, S50, D54, and S96; and
(10) the VL including amino acid mutations at the following positions: D30b, E55, V104, D30, Y30a and G30c; the VH including amino acid mutations at the following positions: N60, P61, I67, I37, A49, S50, D54 and S31.

34. The antigen-binding protein of any one of claims 1-33, comprising an antibody heavy chain constant region.

35. The antigen-binding protein of claim 34, wherein the antibody heavy chain constant region is derived from a human IgG constant region.

36. The antigen-binding protein of claim 35, wherein the human IgG constant region comprises a human IgG l constant region.

37. The antigen-binding protein of claim 36, wherein the human IgG1 constant region comprises one or more amino acid mutations selected from the group consisting of M252Y, S254T and T256E.

38. The antigen-binding protein of claim 37, wherein the human IgG1 constant region comprises any one of amino acid sequences as shown in SEQ ID NOs: 60-61.

39. The antigen-binding protein of any one of claims 1-38, comprising an antibody light chain constant region.

40. The antigen-binding protein of claim 39, wherein the antibody light chain constant region comprises an amino acid sequence as shown in SEQ ID NO: 59.

41. One or more isolated nucleic acid molecules encoding the antigen-binding protein of any one of claims 1-40.

42. A vector comprising the nucleic acid molecules of claim 41.

43. A cell comprising the nucleic acid molecules of claim 41 or the vector of claim 42.

44. A method for preparing the antigen-binding protein of any one of claims 1-40, comprising culturing the cell of claim 43 under a condition allowing expression of the antigen-binding protein of any one of claims 1-40.

45. A pharmaceutical composition comprising the antigen-binding protein of any one of claims 1-40, the nucleic acid molecules of claim 41, the vector of claim 42 and/or the cell of claim 43, and optionally a pharmaceutically acceptable carrier.

46. Use of the antigen-binding protein of any one of claims 1-40, the nucleic acid molecules of claim 41, the vector of claim 42, the cell of claim 43 and/or the pharmaceutical composition of claim 45 in the preparation of a medicament for relieving or treating a disease associated with abnormal level of IgE.

47. A method for alleviating or treating a disease associated with abnormal level of IgE, comprising administering to a subject in need the antigen-binding protein of any one of claims 1-40, the nucleic acid molecules of claim 41, the vector of claim 42, the cell of claim 43 and/or the pharmaceutical composition of claim 45.

48. A polypeptide comprising the antigen-binding protein of any one of claims 1-40.

49. An immunoconjugate comprising the antigen-binding protein of any one of claims 1-40.

50. A kit comprising the antigen-binding protein of any one of claims 1-40, the nucleic acid molecules of claim 41, the vector of claim 42, the cell of claim 43 and/ the pharmaceutical composition of claim 45.
